# EUROPEAN PATENT APPLICATION

(11) **EP 2 631 653 A1**
(43) Date of publication of application: **28.08.2013**
(21) Application number: 12156930.5
(22) Date of filing: 24.02.2012
(51) Int. Cl.: G01N 33/564

(54) **Identification of modulators of binding properties of antibodies reactive with a member of the insulin receptor family**

(71) Applicant: Charité - Universitätsmedizin Berlin, 10117 Berlin (DE); ICI Immunochemical Intelligence GmbH, 10999 Berlin (DE)
(72) Inventor: Schomburg, Lutz, Prof. Dr., 10965 Berlin (DE); Minich, Waldemar, 13599 Berlin (DE); Karasch, Siegmund, 12355 Berlin (DE)
(74) Representative: Gruber, Daniel

(57) **Abstract**

The present invention relates to methods and kits useful in the identification of modulators of the binding properties of antibodies reactive with one or more members of the insulin receptor family selected from the insulin receptor (IR), the insulin-like growth factor 1 receptor (IGF1R), the insulin-like growth factor 2 receptor (IGF2R), the insulin-IGF1 hybrid receptor (IIHR), and the insulin receptor-related receptor (IRRR) and methods for the detection of such antibodies.

SEQUENCE LISTING

<110> CharitÃ© - UniversitÃ¤tsmedizin Berlin
      ICI immunochemical intelligence GmbH

<120> IDENTIFICATION OF MODULATORS OF BINDING PROPERTIES OF ANTIBODIES
REACTIVE WITH A MEMBER OF THE INSULIN RECEPTOR FAMILY

<130> DG011022-P

<160> 20

<170> BiSSAP 1.0

<210> 1
<211> 33
<212> DNA
<213> artificial sequences

<220>
<221> source
<222> 1..33
<223> /mol_type="DNA"
      /note="primer P1"
      /organism="artificial sequences"

<400> 1
gcggaattcg tcaccgacgc caaaaacata aag                                    33


<210> 2
<211> 34
<212> DNA
<213> artificial sequences

<220>
<221> source
<222> 1..34
<223> /mol_type="DNA"
      /note="Primer P2"
      /organism="artificial sequences"

<400> 2
acgggattct tacacggcga tctttccgcc cttc                                   34


<210> 3
<211> 35
<212> DNA
<213> artificial sequences

<220>
<221> source
<222> 1..35
<223> /mol_type="DNA"
      /note="Primer P3"
      /organism="artificial sequences"

<400> 3
acggcggccg catgaagtct ggctccggag gaggg                                  35


<210> 4
<211> 33
<212> DNA

<213> artificial sequences

<220>
<221> source
<222> 1..33
<223> /mol_type="DNA"
      /note="Primer P4"
      /organism="artificial sequences"

<400> 4
acggaattcg caggtcgaag actggggcag cgg                                    33


<210> 5
<211> 1653
<212> DNA
<213> Photinus pyralis

<220>
<221> source
<222> 1..1653
<223> /mol_type="DNA"
      /note="cds"
      /organism="Photinus pyralis"

<400> 5
gtcaccgacg ccaaaaacat aaagaaaggc ccggcgccat tctatccgct ggaagatgga      60

accgctggag agcaactgca taaggctatg aagagatacg ccctggttcc tggaacaatt     120

gcttttacag atgcacatat cgaggtggac atcacttacg ctgagtactt cgaaatgtcc     180

gttcggttgg cagaagctat gaaacgatat gggctgaata caaatcacag aatcgtcgta     240

tgcagtgaaa actctcttca attctttatg ccggtgttgg gcgcgttatt tatcggagtt     300

gcagttgcgc ccgcgaacga catttataat gaacgtgaat tgctcaacag tatgggcatt     360

tcgcagccta ccgtggtgtt cgtttccaaa aaggggttgc aaaaaatttt gaacgtgcaa     420

aaaaagctcc caatcatcca aaaaattatt atcatggatt ctaaaacgga ttaccaggga     480

tttcagtcga tgtacacgtt cgtcacatct catctacctc ccggttttaa tgaatacgat     540

tttgtgccag agtccttcga tagggacaag acaattgcac tgatcatgaa ctcctctgga     600

tctactggtc tgcctaaagg tgtcgctctg cctcatagaa ctgcctgcgt gagattctcg     660

catgccagag atcctatttt tggcaatcaa atcattccgg atactgcgat tttaagtgtt     720

gttccattcc atcacggttt tggaatgttt actacactcg gatatttgat atgtggattt     780

cgagtcgtct taatgtatag atttgaagaa gagctgtttc tgaggagcct tcaggattac     840

aagattcaaa gtgcgctgct ggtgccaacc ctattctcct tcttcgccaa aagcactctg     900

attgacaaat acgatttatc taatttacac gaaattgctt ctggtggcgc tcccctctct     960

aaggaagtcg gggaagcggt tgccaagagg ttccatctgc caggtatcag gcaaggatat    1020

gggctcactg agactacatc agctattctg attacacccg agggggatga taaaccgggc    1080

gcggtcggta aagttgttcc attttttgaa gcgaaggttg tggatctgga taccgggaaa    1140

acgctgggcg ttaatcaaag aggcgaactg tgtgtgagag gtcctatgat tatgtccggt    1200

tatgtaaaca atccggaagc gaccaacgcc ttgattgaca aggatggatg gctacattct 1260

ggagacatag cttactggga cgaagacgaa cacttcttca tcgttgaccg cctgaagtct 1320

ctgattaagt acaaaggcta tcaggtggct cccgctgaat tggaatccat cttgctccaa 1380

caccccaaca tcttcgacgc aggtgtcgca ggtcttcccg acgatgacgc cggtgaactt 1440

cccgccgccg ttgttgtttt ggagcacgga aagacgatga cggaaaaaga gatcgtggat 1500

tacgtcgcca gtcaagtaac aaccgcgaaa aagttgcgcg gaggagttgt gtttgtggac 1560

gaagtaccga aaggtcttac cggaaaactc gacgcaagaa aaatcagaga gatcctcata 1620

aaggccaaga agggcggaaa gatcgccgtg taa 1653

<210> 6
<211> 550
<212> PRT
<213> Photinus pyralis

<220>
<221> SOURCE
<222> 1..550
<223> /mol_type="protein"
/organism="Photinus pyralis"

<400> 6
Val Thr Asp Ala Lys Asn Ile Lys Lys Gly Pro Ala Pro Phe Tyr Pro
1               5                   10                  15
Leu Glu Asp Gly Thr Ala Gly Glu Gln Leu His Lys Ala Met Lys Arg
            20                  25                  30
Tyr Ala Leu Val Pro Gly Thr Ile Ala Phe Thr Asp Ala His Ile Glu
        35                  40                  45
Val Asp Ile Thr Tyr Ala Glu Tyr Phe Glu Met Ser Val Arg Leu Ala
    50                  55                  60
Glu Ala Met Lys Arg Tyr Gly Leu Asn Thr Asn His Arg Ile Val Val
65                  70                  75                  80
Cys Ser Glu Asn Ser Leu Gln Phe Phe Met Pro Val Leu Gly Ala Leu
                85                  90                  95
Phe Ile Gly Val Ala Val Ala Pro Ala Asn Asp Ile Tyr Asn Glu Arg
            100                 105                 110
Glu Leu Leu Asn Ser Met Gly Ile Ser Gln Pro Thr Val Val Phe Val
        115                 120                 125
Ser Lys Lys Gly Leu Gln Lys Ile Leu Asn Val Gln Lys Lys Leu Pro
    130                 135                 140
Ile Ile Gln Lys Ile Ile Ile Met Asp Ser Lys Thr Asp Tyr Gln Gly
145                 150                 155                 160
Phe Gln Ser Met Tyr Thr Phe Val Thr Ser His Leu Pro Pro Gly Phe
                165                 170                 175
Asn Glu Tyr Asp Phe Val Pro Glu Ser Phe Asp Arg Asp Lys Thr Ile
            180                 185                 190
Ala Leu Ile Met Asn Ser Ser Gly Ser Thr Gly Leu Pro Lys Gly Val
        195                 200                 205
Ala Leu Pro His Arg Thr Ala Cys Val Arg Phe Ser His Ala Arg Asp
    210                 215                 220
Pro Ile Phe Gly Asn Gln Ile Ile Pro Asp Thr Ala Ile Leu Ser Val
225                 230                 235                 240
Val Pro Phe His His Gly Phe Gly Met Phe Thr Thr Leu Gly Tyr Leu
                245                 250                 255
Ile Cys Gly Phe Arg Val Val Leu Met Tyr Arg Phe Glu Glu Glu Leu
            260                 265                 270
Phe Leu Arg Ser Leu Gln Asp Tyr Lys Ile Gln Ser Ala Leu Leu Val

275                 280                 285
Pro Thr Leu Phe Ser Phe Phe Ala Lys Ser Thr Leu Ile Asp Lys Tyr
    290                 295                 300
Asp Leu Ser Asn Leu His Glu Ile Ala Ser Gly Gly Ala Pro Leu Ser
305                 310                 315                 320
Lys Glu Val Gly Glu Ala Val Ala Lys Arg Phe His Leu Pro Gly Ile
                325                 330                 335
Arg Gln Gly Tyr Gly Leu Thr Glu Thr Thr Ser Ala Ile Leu Ile Thr
            340                 345                 350
Pro Glu Gly Asp Asp Lys Pro Gly Ala Val Gly Lys Val Val Pro Phe
        355                 360                 365
Phe Glu Ala Lys Val Val Asp Leu Asp Thr Gly Lys Thr Leu Gly Val
    370                 375                 380
Asn Gln Arg Gly Glu Leu Cys Val Arg Gly Pro Met Ile Met Ser Gly
385                 390                 395                 400
Tyr Val Asn Asn Pro Glu Ala Thr Asn Ala Leu Ile Asp Lys Asp Gly
                405                 410                 415
Trp Leu His Ser Gly Asp Ile Ala Tyr Trp Asp Glu Asp Glu His Phe
            420                 425                 430
Phe Ile Val Asp Arg Leu Lys Ser Leu Ile Lys Tyr Lys Gly Tyr Gln
        435                 440                 445
Val Ala Pro Ala Glu Leu Glu Ser Ile Leu Leu Gln His Pro Asn Ile
    450                 455                 460
Phe Asp Ala Gly Val Ala Gly Leu Pro Asp Asp Asp Ala Gly Glu Leu
465                 470                 475                 480
Pro Ala Ala Val Val Val Leu Glu His Gly Lys Thr Met Thr Glu Lys
                485                 490                 495
Glu Ile Val Asp Tyr Val Ala Ser Gln Val Thr Thr Ala Lys Lys Leu
            500                 505                 510
Arg Gly Gly Val Val Phe Val Asp Glu Val Pro Lys Gly Leu Thr Gly
        515                 520                 525
Lys Leu Asp Ala Arg Lys Ile Arg Glu Ile Leu Ile Lys Ala Lys Lys
    530                 535                 540
Gly Gly Lys Ile Ala Val
545                 550

<210> 7
<211> 4104
<212> DNA
<213> Homo sapiens

<220>
<221> source
<222> 1..4104
<223> /mol_type="DNA"
      /note="IGF1R"
      /organism="Homo sapiens"

<400> 7
atgaagtctg gctccggagg agggtccccg acctcgctgt gggggctcct gtttctctcc      60

gccgcgctct cgctctggcc gacgagtgga gaaatctgcg ggccaggcat cgacatccgc     120

aacgactatc agcagctgaa gcgcctggag aactgcacgg tgatcgaggg ctacctccac     180

atcctgctca tctccaaggc cgaggactac cgcagctacc gcttccccaa gctcacggtc     240

attaccgagt acttgctgct gttccgagtg gctggcctcg agagcctcgg agacctcttc     300

cccaacctca cggtcatccg cggctggaaa ctcttctaca actacgccct ggtcatcttc     360

gagatgacca atctcaagga tattgggctt tacaacctga ggaacattac tcggggggcc     420

atcaggattg agaaaaatgc tgacctctgt tacctctcca ctgtggactg gtccctgatc     480

ctggatgcgg tgtccaataa ctacattgtg gggaataagc ccccaaagga atgtggggac 540

ctgtgtccag ggaccatgga ggagaagccg atgtgtgaga agaccaccat caacaatgag 600

tacaactacc gctgctggac cacaaaccgc tgccagaaaa tgtgcccaag cacgtgtggg 660

aagcgggcgt gcaccgagaa caatgagtgc tgccaccccg agtgcctggg cagctgcagc 720

gcgcctgaca acgacacggc ctgtgtagct tgccgccact actactatgc cggtgtctgt 780

gtgcctgcct gcccgcccaa cacctacagg tttgagggct ggcgctgtgt ggaccgtgac 840

ttctgcgcca acatcctcag cgccgagagc agcgactccg aggggtttgt gatccacgac 900

ggcgagtgca tgcaggagtg cccctcgggc ttcatccgca acggcagcca gagcatgtac 960

tgcatccctt gtgaaggtcc ttgcccgaag gtctgtgagg aagaaaagaa aacaaagacc 1020

attgattctg ttacttctgc tcagatgctc caaggatgca ccatcttcaa gggcaatttg 1080

ctcattaaca tccgacgggg gaataacatt gcttcagagc tggagaactt catggggctc 1140

atcgaggtgg tgacgggcta cgtgaagatc cgccattctc atgccttggt ctccttgtcc 1200

ttcctaaaaa accttcgcct catcctagga gaggagcagc tagaagggaa ttactccttc 1260

tacgtcctcg acaaccagaa cttgcagcaa ctgtgggact gggaccaccg caacctgacc 1320

atcaaagcag ggaaaatgta ctttgctttc aatcccaaat tatgtgtttc cgaaatttac 1380

cgcatggagg aagtgacggg gactaaaggg cgccaaagca aaggggacat aaacaccagg 1440

aacaacgggg agagagcctc ctgtgaaagt gacgtcctgc atttcacctc caccaccacg 1500

tcgaagaatc gcatcatcat aacctggcac cggtaccggc cccctgacta cagggatctc 1560

atcagcttca ccgtttacta caaggaagca ccctttaaga atgtcacaga gtatgatggg 1620

caggatgcct gcggctccaa cagctggaac atggtggacg tggacctccc gcccaacaag 1680

gacgtggagc ccggcatctt actacatggg ctgaagccct ggactcagta cgccgtttac 1740

gtcaaggctg tgaccctcac catggtggag aacgaccata tccgtggggc caagagtgag 1800

atcttgtaca ttcgcaccaa tgcttcagtt ccttccattc ccttggacgt tctttcagca 1860

tcgaactcct cttctcagtt aatcgtgaag tggaaccctc cctctctgcc caacggcaac 1920

ctgagttact acattgtgcg ctggcagcgg cagcctcagg acggctacct ttaccggcac 1980

aattactgct ccaaagacaa aatccccatc aggaagtatg ccgacggcac catcgacatt 2040

gaggaggtca cagagaaccc caagactgag gtgtgtggtg gggagaaagg gccttgctgc 2100

gcctgcccca aaactgaagc cgagaagcag gccgagaagg aggaggctga ataccgcaaa 2160

gtctttgaga atttcctgca caactccatc ttcgtgccca gacctgaaag gaagcggaga 2220

gatgtcatgc aagtggccaa caccaccatg tccagccgaa gcaggaacac cacggccgca 2280

gacacctaca acatcaccga cccggaagag ctggagacag agtacccttt ctttgagagc 2340

agagtggata acaaggagag aactgtcatt tctaaccttc ggcctttcac attgtaccgc 2400

atcgatatcc acagctgcaa ccacgaggct gagaagctgg gctgcagcgc ctccaacttc 2460

gtctttgcaa ggactatgcc cgcagaagga gcagatgaca ttcctgggcc agtgacctgg 2520

gagccaaggc ctgaaaactc catcttttta aagtggccgg aacctgagaa tcccaatgga 2580

ttgattctaa tgtatgaaat aaaatacgga tcacaagttg aggatcagcg agaatgtgtg 2640

tccagacagg aatacaggaa gtatggaggg gccaagctaa accggctaaa cccggggaac 2700

tacacagccc ggattcaggc cacatctctc tctgggaatg ggtcgtggac agatcctgtg 2760

ttcttctatg tccaggccaa aacaggatat gaaaacttca tccatctgat catcgctctg 2820

cccgtcgctg tcctgttgat cgtgggaggg ttggtgatta tgctgtacgt cttccataga 2880

aagagaaata acagcaggct ggggaatgga gtgctgtatg cctctgtgaa cccggagtac 2940

ttcagcgctg ctgatgtgta cgttcctgat gagtgggagg tggctcggga gaagatcacc 3000

atgagccggg aacttgggca ggggtcgttt gggatggtct atgaaggagt tgccaagggt 3060

gtggtgaaag atgaacctga aaccagagtg gccattaaaa cagtgaacga ggccgcaagc 3120

atgcgtgaga ggattgagtt tctcaacgaa gcttctgtga tgaaggagtt caattgtcac 3180

catgtggtgc gattgctggg tgtggtgtcc caaggccagc caacactggt catcatggaa 3240

ctgatgacac ggggcgatct caaaagttat ctccggtctc tgaggccaga aatggagaat 3300

aatccagtcc tagcacctcc aagcctgagc aagatgattc agatggccgg agagattgca 3360

gacggcatgg catacctcaa cgccaataag ttcgtccaca gagaccttgc tgcccggaat 3420

tgcatggtag ccgaagattt cacagtcaaa atcggagatt ttggtatgac gcgagatatc 3480

tatgagacag actattaccg gaaaggaggg aaagggctgc tgcccgtgcg ctggatgtct 3540

cctgagtccc tcaaggatgg agtcttcacc acttactcgg acgtctggtc cttcggggtc 3600

gtcctctggg agatcgccac actggccgag cagccctacc agggcttgtc caacgagcaa 3660

gtccttcgct tcgtcatgga gggcggcctt ctggacaagc cagacaactg tcctgacatg 3720

ctgtttgaac tgatgcgcat gtgctggcag tataacccca agatgaggcc ttccttcctg 3780

gagatcatca gcagcatcaa agaggagatg gagcctggct tccgggaggt ctccttctac 3840

tacagcgagg agaacaagct gcccgagccg gaggagctgg acctggagcc agagaacatg 3900

gagagcgtcc ccctggaccc ctcggcctcc tcgtcctccc tgccactgcc cgacagacac 3960

tcaggacaca aggccgagaa cggccccggc cctggggtgc tggtcctccg cgccagcttc 4020

gacgagagac agccttacgc ccacatgaac gggggccgca agaacgagcg ggccttgccg 4080

ctgccccagt cttcgacctg ctga 4104

<210> 8
<211> 1367
<212> PRT
<213> Homo sapiens

<220>
<221> SOURCE

<222> 1..1367
<223> /mol_type="protein"
      /note="IGF1R"
      /organism="Homo sapiens"

<400> 8

<210> 9
<211> 5760
<212> DNA
<213> artificial sequences

<220>
<221> source
<222> 1..5760
<223> /mol_type="DNA"
      /note="hIGF1R-luc fusion"
      /organism="artificial sequences"

<400> 9
atgaagtctg gctccggagg agggtccccg acctcgctgt gggggctcct gtttctctcc      60

gccgcgctct cgctctggcc gacgagtgga gaaatctgcg ggccaggcat cgacatccgc     120

aacgactatc agcagctgaa gcgcctggag aactgcacgg tgatcgaggg ctacctccac 180
atcctgctca tctccaaggc cgaggactac cgcagctacc gcttccccaa gctcacggtc 240
attaccgagt acttgctgct gttccgagtg gctggcctcg agagcctcgg agacctcttc 300
cccaacctca cggtcatccg cggctggaaa ctcttctaca actacgccct ggtcatcttc 360
gagatgacca atctcaagga tattgggctt tacaacctga ggaacattac tcggggggcc 420
atcaggattg agaaaaatgc tgacctctgt tacctctcca ctgtggactg gtccctgatc 480
ctggatgcgg tgtccaataa ctacattgtg gggaataagc ccccaaagga atgtggggac 540
ctgtgtccag ggaccatgga ggagaagccg atgtgtgaga agaccaccat caacaatgag 600
tacaactacc gctgctggac cacaaaccgc tgccagaaaa tgtgcccaag cacgtgtggg 660
aagcgggcgt gcaccgagaa caatgagtgc tgccaccccg agtgcctggg cagctgcagc 720
gcgcctgaca acgacacggc ctgtgtagct tgccgccact actactatgc cggtgtctgt 780
gtgcctgcct gcccgcccaa cacctacagg tttgagggct ggcgctgtgt ggaccgtgac 840
ttctgcgcca acatcctcag cgccgagagc agcgactccg aggggtttgt gatccacgac 900
ggcgagtgca tgcaggagtg cccctcgggc ttcatccgca acggcagcca gagcatgtac 960
tgcatccctt gtgaaggtcc ttgcccgaag gtctgtgagg aagaaaagaa aacaaagacc 1020
attgattctg ttacttctgc tcagatgctc caaggatgca ccatcttcaa gggcaatttg 1080
ctcattaaca tccgacgggg gaataacatt gcttcagagc tggagaactt catggggctc 1140
atcgaggtgg tgacgggcta cgtgaagatc cgccattctc atgccttggt ctccttgtcc 1200
ttcctaaaaa accttcgcct catcctagga gaggagcagc tagaagggaa ttactccttc 1260
tacgtcctcg acaaccagaa cttgcagcaa ctgtgggact gggaccaccg caacctgacc 1320
atcaaagcag ggaaaatgta ctttgctttc aatcccaaat tatgtgtttc cgaaatttac 1380
cgcatggagg aagtgacggg gactaaaggg cgccaaagca aaggggacat aaacaccagg 1440
aacaacgggg agagagcctc ctgtgaaagt gacgtcctgc atttcacctc caccaccacg 1500
tcgaagaatc gcatcatcat aacctggcac cggtaccggc cccctgacta cagggatctc 1560
atcagcttca ccgtttacta caaggaagca ccctttaaga atgtcacaga gtatgatggg 1620
caggatgcct gcggctccaa cagctggaac atggtggacg tggacctccc gcccaacaag 1680
gacgtggagc ccggcatctt actacatggg ctgaagccct ggactcagta cgccgtttac 1740
gtcaaggctg tgaccctcac catggtggag aacgaccata tccgtggggc caagagtgag 1800
atcttgtaca ttcgcaccaa tgcttcagtt ccttccattc ccttggacgt tctttcagca 1860
tcgaactcct cttctcagtt aatcgtgaag tggaaccctc cctctctgcc caacggcaac 1920
ctgagttact acattgtgcg ctggcagcgg cagcctcagg acggctacct ttaccggcac 1980
aattactgct ccaaagacaa aatccccatc aggaagtatg ccgacggcac catcgacatt 2040

gaggaggtca cagagaaccc caagactgag gtgtgtggtg gggagaaagg gccttgctgc 2100

gcctgcccca aaactgaagc cgagaagcag gccgagaagg aggaggctga ataccgcaaa 2160

gtctttgaga atttcctgca caactccatc ttcgtgccca gacctgaaag gaagcggaga 2220

gatgtcatgc aagtggccaa caccaccatg tccagccgaa gcaggaacac cacggccgca 2280

gacacctaca acatcaccga cccggaagag ctggagacag agtacccttt ctttgagagc 2340

agagtggata acaaggagag aactgtcatt tctaaccttc ggcctttcac attgtaccgc 2400

atcgatatcc acagctgcaa ccacgaggct gagaagctgg gctgcagcgc ctccaacttc 2460

gtctttgcaa ggactatgcc cgcagaagga gcagatgaca ttcctgggcc agtgacctgg 2520

gagccaaggc ctgaaaactc catcttttta aagtggccgg aacctgagaa tcccaatgga 2580

ttgattctaa tgtatgaaat aaaatacgga tcacaagttg aggatcagcg agaatgtgtg 2640

tccagacagg aatacaggaa gtatggaggg gccaagctaa accggctaaa cccggggaac 2700

tacacagccc ggattcaggc cacatctctc tctgggaatg ggtcgtggac agatcctgtg 2760

ttcttctatg tccaggccaa aacaggatat gaaaacttca tccatctgat catcgctctg 2820

cccgtcgctg tcctgttgat cgtgggaggg ttggtgatta tgctgtacgt cttccataga 2880

aagagaaata acagcaggct ggggaatgga gtgctgtatg cctctgtgaa cccggagtac 2940

ttcagcgctg ctgatgtgta cgttcctgat gagtgggagg tggctcggga gaagatcacc 3000

atgagccggg aacttgggca ggggtcgttt gggatggtct atgaaggagt tgccaagggt 3060

gtggtgaaag atgaacctga aaccagagtg gccattaaaa cagtgaacga ggccgcaagc 3120

atgcgtgaga ggattgagtt tctcaacgaa gcttctgtga tgaaggagtt caattgtcac 3180

catgtggtgc gattgctggg tgtggtgtcc caaggccagc caacactggt catcatggaa 3240

ctgatgacac ggggcgatct caaaagttat ctccggtctc tgaggccaga aatggagaat 3300

aatccagtcc tagcacctcc aagcctgagc aagatgattc agatggccgg agagattgca 3360

gacggcatgg catacctcaa cgccaataag ttcgtccaca gagaccttgc tgcccggaat 3420

tgcatggtag ccgaagattt cacagtcaaa atcggagatt ttggtatgac gcgagatatc 3480

tatgagacag actattaccg gaaaggaggg aaagggctgc tgcccgtgcg ctggatgtct 3540

cctgagtccc tcaaggatgg agtcttcacc acttactcgg acgtctggtc cttcggggtc 3600

gtcctctggg agatcgccac actggccgag cagccctacc agggcttgtc caacgagcaa 3660

gtccttcgct tcgtcatgga gggcggcctt ctggacaagc cagacaactg tcctgacatg 3720

ctgtttgaac tgatgcgcat gtgctggcag tataacccca agatgaggcc ttccttcctg 3780

gagatcatca gcagcatcaa agaggagatg gagcctggct tccgggaggt ctccttctac 3840

tacagcgagg agaacaagct gcccgagccg gaggagctgg acctggagcc agagaacatg 3900

gagagcgtcc ccctggaccc ctcggcctcc tcgtcctccc tgccactgcc cgacagacac 3960

tcaggacaca aggccgagaa cggccccggc cctggggtgc tggtcctccg cgccagcttc 4020

gacgagagac agccttacgc ccacatgaac gggggccgca agaacgagcg ggccttgccg 4080

ctgccccagt cttcgacctg cgaattcgtc accgacgcca aaaacataaa gaaaggcccg 4140

gcgccattct atccgctgga agatggaacc gctggagagc aactgcataa ggctatgaag 4200

agatacgccc tggttcctgg aacaattgct tttacagatg cacatatcga ggtggacatc 4260

acttacgctg agtacttcga aatgtccgtt cggttggcag aagctatgaa acgatatggg 4320

ctgaatacaa atcacagaat cgtcgtatgc agtgaaaact ctcttcaatt ctttatgccg 4380

gtgttgggcg cgttatttat cggagttgca gttgcgcccg cgaacgacat ttataatgaa 4440

cgtgaattgc tcaacagtat gggcatttcg cagcctaccg tggtgttcgt ttccaaaaag 4500

gggttgcaaa aaattttgaa cgtgcaaaaa aagctcccaa tcatccaaaa aattattatc 4560

atggattcta aaacggatta ccagggattt cagtcgatgt acacgttcgt cacatctcat 4620

ctacctcccg gttttaatga atacgatttt gtgccagagt ccttcgatag ggacaagaca 4680

attgcactga tcatgaactc ctctggatct actggtctgc ctaaaggtgt cgctctgcct 4740

catagaactg cctgcgtgag attctcgcat gccagagatc ctatttttgg caatcaaatc 4800

attccggata ctgcgatttt aagtgttgtt ccattccatc acggttttgg aatgtttact 4860

acactcggat atttgatatg tggatttcga gtcgtcttaa tgtatagatt tgaagaagag 4920

ctgtttctga ggagccttca ggattacaag attcaaagtg cgctgctggt gccaacccta 4980

ttctccttct tcgccaaaag cactctgatt gacaaatacg atttatctaa tttacacgaa 5040

attgcttctg gtggcgctcc cctctctaag gaagtcgggg aagcggttgc caagaggttc 5100

catctgccag gtatcaggca aggatatggg ctcactgaga ctacatcagc tattctgatt 5160

acacccgagg gggatgataa accgggcgcg gtcggtaaag ttgttccatt ttttgaagcg 5220

aaggttgtgg atctggatac cgggaaaacg ctgggcgtta atcaaagagg cgaactgtgt 5280

gtgagaggtc ctatgattat gtccggttat gtaaacaatc cggaagcgac caacgccttg 5340

attgacaagg atggatggct acattctgga gacatagctt actgggacga agacgaacac 5400

ttcttcatcg ttgaccgcct gaagtctctg attaagtaca aaggctatca ggtggctccc 5460

gctgaattgg aatccatctt gctccaacac cccaacatct tcgacgcagg tgtcgcaggt 5520

cttcccgacg atgacgccgg tgaacttccc gccgccgttg ttgttttgga gcacggaaag 5580

acgatgacgg aaaaagagat cgtggattac gtcgccagtc aagtaacaac cgcgaaaaag 5640

ttgcgcggag gagttgtgtt tgtggacgaa gtaccgaaag gtcttaccgg aaaactcgac 5700

gcaagaaaaa tcagagagat cctcataaag gccaagaagg gcggaaagat cgccgtgtaa 5760

<210> 10
<211> 1919
<212> PRT
<213> artificial sequences

<220>
<221> SOURCE
<222> 1..1919
<223> /mol_type="protein"
      /note="hIGF1R-luc fusion protein"
      /organism="artificial sequences"

<400> 10
Met Lys Ser Gly Ser Gly Gly Gly Ser Pro Thr Ser Leu Trp Gly Leu
1               5                   10                  15
Leu Phe Leu Ser Ala Ala Leu Ser Leu Trp Pro Thr Ser Gly Glu Ile
            20                  25                  30
Cys Gly Pro Gly Ile Asp Ile Arg Asn Asp Tyr Gln Gln Leu Lys Arg
        35                  40                  45
Leu Glu Asn Cys Thr Val Ile Glu Gly Tyr Leu His Ile Leu Leu Ile
    50                  55                  60
Ser Lys Ala Glu Asp Tyr Arg Ser Tyr Arg Phe Pro Lys Leu Thr Val
65                  70                  75                  80
Ile Thr Glu Tyr Leu Leu Leu Phe Arg Val Ala Gly Leu Glu Ser Leu
                85                  90                  95
Gly Asp Leu Phe Pro Asn Leu Thr Val Ile Arg Gly Trp Lys Leu Phe
            100                 105                 110
Tyr Asn Tyr Ala Leu Val Ile Phe Glu Met Thr Asn Leu Lys Asp Ile
        115                 120                 125
Gly Leu Tyr Asn Leu Arg Asn Ile Thr Arg Gly Ala Ile Arg Ile Glu
    130                 135                 140
Lys Asn Ala Asp Leu Cys Tyr Leu Ser Thr Val Asp Trp Ser Leu Ile
145                 150                 155                 160
Leu Asp Ala Val Ser Asn Asn Tyr Ile Val Gly Asn Lys Pro Pro Lys
                165                 170                 175
Glu Cys Gly Asp Leu Cys Pro Gly Thr Met Glu Glu Lys Pro Met Cys
            180                 185                 190
Glu Lys Thr Thr Ile Asn Asn Glu Tyr Asn Tyr Arg Cys Trp Thr Thr
        195                 200                 205
Asn Arg Cys Gln Lys Met Cys Pro Ser Thr Cys Gly Lys Arg Ala Cys
    210                 215                 220
Thr Glu Asn Asn Glu Cys Cys His Pro Glu Cys Leu Gly Ser Cys Ser
225                 230                 235                 240
Ala Pro Asp Asn Asp Thr Ala Cys Val Ala Cys Arg His Tyr Tyr Tyr
                245                 250                 255
Ala Gly Val Cys Val Pro Ala Cys Pro Pro Asn Thr Tyr Arg Phe Glu
            260                 265                 270
Gly Trp Arg Cys Val Asp Arg Asp Phe Cys Ala Asn Ile Leu Ser Ala
        275                 280                 285
Glu Ser Ser Asp Ser Glu Gly Phe Val Ile His Asp Gly Glu Cys Met
    290                 295                 300
Gln Glu Cys Pro Ser Gly Phe Ile Arg Asn Gly Ser Gln Ser Met Tyr
305                 310                 315                 320
Cys Ile Pro Cys Glu Gly Pro Cys Pro Lys Val Cys Glu Glu Glu Lys
                325                 330                 335
Lys Thr Lys Thr Ile Asp Ser Val Thr Ser Ala Gln Met Leu Gln Gly
            340                 345                 350
Cys Thr Ile Phe Lys Gly Asn Leu Leu Ile Asn Ile Arg Arg Gly Asn
        355                 360                 365
Asn Ile Ala Ser Glu Leu Glu Asn Phe Met Gly Leu Ile Glu Val Val
    370                 375                 380
Thr Gly Tyr Val Lys Ile Arg His Ser His Ala Leu Val Ser Leu Ser
385                 390                 395                 400
Phe Leu Lys Asn Leu Arg Leu Ile Leu Gly Glu Glu Gln Leu Glu Gly
                405                 410                 415
Asn Tyr Ser Phe Tyr Val Leu Asp Asn Gln Asn Leu Gln Gln Leu Trp
            420                 425                 430
Asp Trp Asp His Arg Asn Leu Thr Ile Lys Ala Gly Lys Met Tyr Phe
        435                 440                 445
Ala Phe Asn Pro Lys Leu Cys Val Ser Glu Ile Tyr Arg Met Glu Glu

450                 455                 460
Val Thr Gly Thr Lys Gly Arg Gln Ser Lys Gly Asp Ile Asn Thr Arg
465                 470                 475                 480
Asn Asn Gly Glu Arg Ala Ser Cys Glu Ser Asp Val Leu His Phe Thr
                485                 490                 495
Ser Thr Thr Thr Ser Lys Asn Arg Ile Ile Ile Thr Trp His Arg Tyr
            500                 505                 510
Arg Pro Pro Asp Tyr Arg Asp Leu Ile Ser Phe Thr Val Tyr Tyr Lys
        515                 520                 525
Glu Ala Pro Phe Lys Asn Val Thr Glu Tyr Asp Gly Gln Asp Ala Cys
    530                 535                 540
Gly Ser Asn Ser Trp Asn Met Val Asp Val Asp Leu Pro Pro Asn Lys
545                 550                 555                 560
Asp Val Glu Pro Gly Ile Leu Leu His Gly Leu Lys Pro Trp Thr Gln
                565                 570                 575
Tyr Ala Val Tyr Val Lys Ala Val Thr Leu Thr Met Val Glu Asn Asp
            580                 585                 590
His Ile Arg Gly Ala Lys Ser Glu Ile Leu Tyr Ile Arg Thr Asn Ala
        595                 600                 605
Ser Val Pro Ser Ile Pro Leu Asp Val Leu Ser Ala Ser Asn Ser Ser
    610                 615                 620
Ser Gln Leu Ile Val Lys Trp Asn Pro Pro Ser Leu Pro Asn Gly Asn
625                 630                 635                 640
Leu Ser Tyr Tyr Ile Val Arg Trp Gln Arg Gln Pro Gln Asp Gly Tyr
                645                 650                 655
Leu Tyr Arg His Asn Tyr Cys Ser Lys Asp Lys Ile Pro Ile Arg Lys
            660                 665                 670
Tyr Ala Asp Gly Thr Ile Asp Ile Glu Glu Val Thr Glu Asn Pro Lys
        675                 680                 685
Thr Glu Val Cys Gly Gly Glu Lys Gly Pro Cys Cys Ala Cys Pro Lys
    690                 695                 700
Thr Glu Ala Glu Lys Gln Ala Glu Lys Glu Glu Ala Glu Tyr Arg Lys
705                 710                 715                 720
Val Phe Glu Asn Phe Leu His Asn Ser Ile Phe Val Pro Arg Pro Glu
                725                 730                 735
Arg Lys Arg Arg Asp Val Met Gln Val Ala Asn Thr Thr Met Ser Ser
            740                 745                 750
Arg Ser Arg Asn Thr Thr Ala Ala Asp Thr Tyr Asn Ile Thr Asp Pro
        755                 760                 765
Glu Glu Leu Glu Thr Glu Tyr Pro Phe Phe Glu Ser Arg Val Asp Asn
    770                 775                 780
Lys Glu Arg Thr Val Ile Ser Asn Leu Arg Pro Phe Thr Leu Tyr Arg
785                 790                 795                 800
Ile Asp Ile His Ser Cys Asn His Glu Ala Glu Lys Leu Gly Cys Ser
                805                 810                 815
Ala Ser Asn Phe Val Phe Ala Arg Thr Met Pro Ala Glu Gly Ala Asp
            820                 825                 830
Asp Ile Pro Gly Pro Val Thr Trp Glu Pro Arg Pro Glu Asn Ser Ile
        835                 840                 845
Phe Leu Lys Trp Pro Glu Pro Glu Asn Pro Asn Gly Leu Ile Leu Met
    850                 855                 860
Tyr Glu Ile Lys Tyr Gly Ser Gln Val Glu Asp Gln Arg Glu Cys Val
865                 870                 875                 880
Ser Arg Gln Glu Tyr Arg Lys Tyr Gly Gly Ala Lys Leu Asn Arg Leu
                885                 890                 895
Asn Pro Gly Asn Tyr Thr Ala Arg Ile Gln Ala Thr Ser Leu Ser Gly
            900                 905                 910
Asn Gly Ser Trp Thr Asp Pro Val Phe Phe Tyr Val Gln Ala Lys Thr
        915                 920                 925
Gly Tyr Glu Asn Phe Ile His Leu Ile Ile Ala Leu Pro Val Ala Val
    930                 935                 940
Leu Leu Ile Val Gly Gly Leu Val Ile Met Leu Tyr Val Phe His Arg
945                 950                 955                 960
Lys Arg Asn Asn Ser Arg Leu Gly Asn Gly Val Leu Tyr Ala Ser Val
                965                 970                 975

Asn Pro Glu Tyr Phe Ser Ala Ala Asp Val Tyr Val Pro Asp Glu Trp
            980                 985                 990
Glu Val Ala Arg Glu Lys Ile Thr Met Ser Arg Glu Leu Gly Gln Gly
        995                 1000                1005
Ser Phe Gly Met Val Tyr Glu Gly Val Ala Lys Gly Val Val Lys Asp
    1010                1015                1020
Glu Pro Glu Thr Arg Val Ala Ile Lys Thr Val Asn Glu Ala Ala Ser
1025                1030                1035                1040
Met Arg Glu Arg Ile Glu Phe Leu Asn Glu Ala Ser Val Met Lys Glu
                1045                1050                1055
Phe Asn Cys His His Val Val Arg Leu Leu Gly Val Val Ser Gln Gly
            1060                1065                1070
Gln Pro Thr Leu Val Ile Met Glu Leu Met Thr Arg Gly Asp Leu Lys
        1075                1080                1085
Ser Tyr Leu Arg Ser Leu Arg Pro Glu Met Glu Asn Asn Pro Val Leu
    1090                1095                1100
Ala Pro Pro Ser Leu Ser Lys Met Ile Gln Met Ala Gly Glu Ile Ala
1105                1110                1115                1120
Asp Gly Met Ala Tyr Leu Asn Ala Asn Lys Phe Val His Arg Asp Leu
                1125                1130                1135
Ala Ala Arg Asn Cys Met Val Ala Glu Asp Phe Thr Val Lys Ile Gly
            1140                1145                1150
Asp Phe Gly Met Thr Arg Asp Ile Tyr Glu Thr Asp Tyr Tyr Arg Lys
        1155                1160                1165
Gly Gly Lys Gly Leu Leu Pro Val Arg Trp Met Ser Pro Glu Ser Leu
    1170                1175                1180
Lys Asp Gly Val Phe Thr Thr Tyr Ser Asp Val Trp Ser Phe Gly Val
1185                1190                1195                1200
Val Leu Trp Glu Ile Ala Thr Leu Ala Glu Gln Pro Tyr Gln Gly Leu
                1205                1210                1215
Ser Asn Glu Gln Val Leu Arg Phe Val Met Glu Gly Gly Leu Leu Asp
            1220                1225                1230
Lys Pro Asp Asn Cys Pro Asp Met Leu Phe Glu Leu Met Arg Met Cys
        1235                1240                1245
Trp Gln Tyr Asn Pro Lys Met Arg Pro Ser Phe Leu Glu Ile Ile Ser
    1250                1255                1260
Ser Ile Lys Glu Glu Met Glu Pro Gly Phe Arg Glu Val Ser Phe Tyr
1265                1270                1275                1280
Tyr Ser Glu Glu Asn Lys Leu Pro Glu Pro Glu Glu Leu Asp Leu Glu
                1285                1290                1295
Pro Glu Asn Met Glu Ser Val Pro Leu Asp Pro Ser Ala Ser Ser Ser
            1300                1305                1310
Ser Leu Pro Leu Pro Asp Arg His Ser Gly His Lys Ala Glu Asn Gly
        1315                1320                1325
Pro Gly Pro Gly Val Leu Val Leu Arg Ala Ser Phe Asp Glu Arg Gln
    1330                1335                1340
Pro Tyr Ala His Met Asn Gly Gly Arg Lys Asn Glu Arg Ala Leu Pro
1345                1350                1355                1360
Leu Pro Gln Ser Ser Thr Cys Glu Phe Val Thr Asp Ala Lys Asn Ile
                1365                1370                1375
Lys Lys Gly Pro Ala Pro Phe Tyr Pro Leu Glu Asp Gly Thr Ala Gly
            1380                1385                1390
Glu Gln Leu His Lys Ala Met Lys Arg Tyr Ala Leu Val Pro Gly Thr
        1395                1400                1405
Ile Ala Phe Thr Asp Ala His Ile Glu Val Asp Ile Thr Tyr Ala Glu
    1410                1415                1420
Tyr Phe Glu Met Ser Val Arg Leu Ala Glu Ala Met Lys Arg Tyr Gly
1425                1430                1435                1440
Leu Asn Thr Asn His Arg Ile Val Val Cys Ser Glu Asn Ser Leu Gln
                1445                1450                1455
Phe Phe Met Pro Val Leu Gly Ala Leu Phe Ile Gly Val Ala Val Ala
            1460                1465                1470
Pro Ala Asn Asp Ile Tyr Asn Glu Arg Glu Leu Leu Asn Ser Met Gly
        1475                1480                1485
Ile Ser Gln Pro Thr Val Val Phe Val Ser Lys Lys Gly Leu Gln Lys

1490                1495                1500
Ile Leu Asn Val Gln Lys Lys Leu Pro Ile Ile Gln Lys Ile Ile Ile
1505                1510                1515                1520
Met Asp Ser Lys Thr Asp Tyr Gln Gly Phe Gln Ser Met Tyr Thr Phe
                1525                1530                1535
Val Thr Ser His Leu Pro Pro Gly Phe Asn Glu Tyr Asp Phe Val Pro
            1540                1545                1550
Glu Ser Phe Asp Arg Asp Lys Thr Ile Ala Leu Ile Met Asn Ser Ser
        1555                1560                1565
Gly Ser Thr Gly Leu Pro Lys Gly Val Ala Leu Pro His Arg Thr Ala
    1570                1575                1580
Cys Val Arg Phe Ser His Ala Arg Asp Pro Ile Phe Gly Asn Gln Ile
1585                1590                1595                1600
Ile Pro Asp Thr Ala Ile Leu Ser Val Val Pro Phe His His Gly Phe
                1605                1610                1615
Gly Met Phe Thr Thr Leu Gly Tyr Leu Ile Cys Gly Phe Arg Val Val
            1620                1625                1630
Leu Met Tyr Arg Phe Glu Glu Glu Leu Phe Leu Arg Ser Leu Gln Asp
        1635                1640                1645
Tyr Lys Ile Gln Ser Ala Leu Leu Val Pro Thr Leu Phe Ser Phe Phe
    1650                1655                1660
Ala Lys Ser Thr Leu Ile Asp Lys Tyr Asp Leu Ser Asn Leu His Glu
1665                1670                1675                1680
Ile Ala Ser Gly Gly Ala Pro Leu Ser Lys Glu Val Gly Glu Ala Val
                1685                1690                1695
Ala Lys Arg Phe His Leu Pro Gly Ile Arg Gln Gly Tyr Gly Leu Thr
            1700                1705                1710
Glu Thr Thr Ser Ala Ile Leu Ile Thr Pro Glu Gly Asp Asp Lys Pro
        1715                1720                1725
Gly Ala Val Gly Lys Val Val Pro Phe Phe Glu Ala Lys Val Val Asp
    1730                1735                1740
Leu Asp Thr Gly Lys Thr Leu Gly Val Asn Gln Arg Gly Glu Leu Cys
1745                1750                1755                1760
Val Arg Gly Pro Met Ile Met Ser Gly Tyr Val Asn Asn Pro Glu Ala
                1765                1770                1775
Thr Asn Ala Leu Ile Asp Lys Asp Gly Trp Leu His Ser Gly Asp Ile
            1780                1785                1790
Ala Tyr Trp Asp Glu Asp Glu His Phe Phe Ile Val Asp Arg Leu Lys
        1795                1800                1805
Ser Leu Ile Lys Tyr Lys Gly Tyr Gln Val Ala Pro Ala Glu Leu Glu
    1810                1815                1820
Ser Ile Leu Leu Gln His Pro Asn Ile Phe Asp Ala Gly Val Ala Gly
1825                1830                1835                1840
Leu Pro Asp Asp Asp Ala Gly Glu Leu Pro Ala Ala Val Val Val Leu
                1845                1850                1855
Glu His Gly Lys Thr Met Thr Glu Lys Glu Ile Val Asp Tyr Val Ala
            1860                1865                1870
Ser Gln Val Thr Thr Ala Lys Lys Leu Arg Gly Gly Val Val Phe Val
        1875                1880                1885
Asp Glu Val Pro Lys Gly Leu Thr Gly Lys Leu Asp Ala Arg Lys Ile
    1890                1895                1900
Arg Glu Ile Leu Ile Lys Ala Lys Lys Gly Gly Lys Ile Ala Val
1905                1910                1915

<210> 11
<211> 4113
<212> DNA
<213> Homo sapiens

<220>
<221> source
<222> 1..4113
<223> /mol_type="DNA"
      /note="cDNA insulin receptor"
      /organism="Homo sapiens"

<400> 11
atgggcaccg ggggccggcg gggggcggcg gccgcgccgc tgctggtggc ggtggccgcg 60

ctgctactgg gcgccgcggg ccacctgtac cccggagagg tgtgtcccgg catggatatc 120

cggaacaacc tcactaggtt gcatgagctg gagaattgct ctgtcatcga aggacacttg 180

cagatactct tgatgttcaa aacgaggccc gaagatttcc gagacctcag tttccccaaa 240

ctcatcatga tcactgatta cttgctgctc ttccgggtct atgggctcga gagcctgaag 300

gacctgttcc ccaacctcac ggtcatccgg ggatcacgac tgttctttaa ctacgcgctg 360

gtcatcttcg agatggttca cctcaaggaa ctcggcctct acaacctgat gaacatcacc 420

cggggttctg tccgcatcga gaagaacaat gagctctgtt acttggccac tatcgactgg 480

tcccgtatcc tggattccgt ggaggataat tacatcgtgt tgaacaaaga tgacaacgag 540

gagtgtggag acatctgtcc gggtaccgcg aagggcaaga ccaactgccc cgccaccgtc 600

atcaacgggc agtttgtcga acgatgttgg actcatagtc actgccagaa agtttgcccg 660

accatctgta agtcacacgg ctgcaccgcc gaaggcctct gttgccacag cgagtgcctg 720

ggcaactgtt ctcagcccga cgaccccacc aagtgcgtgg cctgccgcaa cttctacctg 780

gacggcaggt gtgtggagac ctgcccgccc ccgtactacc acttccagga ctggcgctgt 840

gtgaacttca gcttctgcca ggacctgcac cacaaatgca agaactcgcg gaggcagggc 900

tgccaccagt acgtcattca caacaacaag tgcatccctg agtgtccctc cgggtacacg 960

atgaattcca gcaacttgct gtgcacccca tgcctgggtc cctgtcccaa ggtgtgccac 1020

ctcctagaag gcgagaagac catcgactcg gtgacgtctg cccaggagct ccgaggatgc 1080

accgtcatca acgggagtct gatcatcaac attcgaggag gcaacaatct ggcagctgag 1140

ctagaagcca acctcggcct cattgaagaa atttcagggt atctaaaaat ccgccgatcc 1200

tacgctctgg tgtcactttc cttcttccgg aagttacgtc tgattcgagg agagaccttg 1260

gaaattggga actactcctt ctatgccttg gacaaccaga acctaaggca gctctgggac 1320

tggagcaaac acaacctcac catcactcag gggaaactct tcttccacta taaccccaaa 1380

ctctgcttgt cagaaatcca caagatggaa gaagtttcag gaaccaaggg gcgccaggag 1440

agaaacgaca ttgccctgaa gaccaatggg gaccaggcat cctgtgaaaa tgagttactt 1500

aaattttctt acattcggac atcttttgac aagatcttgc tgagatggga gccgtactgg 1560

ccccccgact tccgagacct cttggggttc atgctgttct acaaagaggc cccttatcag 1620

aatgtgacgg agttcgacgg gcaggatgcg tgtggttcca acagttggac ggtggtagac 1680

attgacccac ccctgaggtc caacgacccc aaatcacaga accacccagg gtggctgatg 1740

cggggtctca agccctggac ccagtatgcc atctttgtga agaccctggt caccttttcg 1800

gatgaacgcc ggacctatgg ggccaagagt gacatcattt atgtccagac agatgccacc 1860

aacccctctg tgcccctgga tccaatctca gtgtctaact catcatccca gattattctg 1920

aagtggaaac caccctccga ccccaatggc aacatcaccc actacctggt tttctgggag 1980

aggcaggcgg aagacagtga gctgttcgag ctggattatt gcctcaaagg gctgaagctg 2040

ccctcgagga cctggtctcc accattcgag tctgaagatt ctcagaagca caaccagagt 2100

gagtatgagg attcggccgg cgaatgctgc tcctgtccaa agacagactc tcagatcctg 2160

aaggagctgg aggagtcctc gtttaggaag acgtttgagg attacctgca caacgtggtt 2220

ttcgtcccca ggccatctcg gaaacgcagg tcccttggcg atgttgggaa tgtgacggtg 2280

gccgtgccca cggtggcagc tttccccaac acttcctcga ccagcgtgcc cacgagtccg 2340

gaggagcaca ggccttttga gaaggtggtg aacaaggagt cgctggtcat ctccggcttg 2400

cgacacttca cgggctatcg catcgagctg caggcttgca accaggacac ccctgaggaa 2460

cggtgcagtg tggcagccta cgtcagtgcg aggaccatgc ctgaagccaa ggctgatgac 2520

attgttggcc ctgtgacgca tgaaatcttt gagaacaacg tcgtccactt gatgtggcag 2580

gagccgaagg agcccaatgg tctgatcgtg ctgtatgaag tgagttatcg gcgatatggt 2640

gatgaggagc tgcatctctg cgtctcccgc aagcacttcg ctctggaacg gggctgcagg 2700

ctgcgtgggc tgtcaccggg gaactacagc gtgcgaatcc gggccacctc ccttgcgggc 2760

aacggctctt ggacggaacc cacctatttc tacgtgacag actatttaga cgtcccgtca 2820

aatattgcaa aaattatcat cggccccctc atctttgtct ttctcttcag tgttgtgatt 2880

ggaagtattt atctattcct gagaaagagg cagccagatg ggccgctggg accgctttac 2940

gcttcttcaa accctgagta tctcagtgcc agtgatgtgt ttccatgctc tgtgtacgtg 3000

ccggacgagt gggaggtgtc tcgagagaag atcaccctcc ttcgagagct ggggcagggc 3060

tccttcggca tggtgtatga gggcaatgcc agggacatca tcaagggtga ggcagagacc 3120

cgcgtggcgg tgaagacggt caacgagtca gccagtctcc gagagcggat tgagttcctc 3180

aatgaggcct cggtcatgaa gggcttcacc tgccatcatg tggtgcgcct cctgggagtg 3240

gtgtccaagg gccagcccac gctggtggtg atggagctga tggctcacgg agacctgaag 3300

agctacctcc gttctctgcg gccagaggct gagaataatc ctggccgccc tccccctacc 3360

cttcaagaga tgattcagat ggcggcagag attgctgacg ggatggccta cctgaacgcc 3420

aagaagtttg tgcatcggga cctggcagcg agaaactgca tggtcgccca tgattttact 3480

gtcaaaattg gagactttgg aatgaccaga gacatctatg aaacggatta ctaccggaaa 3540

gggggcaagg gtctgctccc tgtacggtgg atggcaccgg agtccctgaa ggatggggtc 3600

ttcaccactt cttctgacat gtggtccttt ggcgtggtcc tttgggaaat caccagcttg 3660

gcagaacagc cttaccaagg cctgtctaat gaacaggtgt tgaaatttgt catggatgga 3720

gggtatctgg atcaacccga caactgtcca gagagagtca ctgacctcat gcgcatgtgc 3780

tggcaattca accccaagat gaggccaacc ttcctggaga ttgtcaacct gctcaaggac 3840

gacctgcacc ccagctttcc agaggtgtcg ttcttccaca gcgaggagaa caaggctccc     3900

gagagtgagg agctggagat ggagtttgag gacatggaga atgtgcccct ggaccgttcc     3960

tcgcactgtc agagggagga ggcggggggc cgggatggag ggtcctcgct gggtttcaag     4020

cggagctacg aggaacacat cccttacaca cacatgaacg gaggcaagaa aaacgggcgg     4080

attctgacct tgcctcggtc caatccttcc taa                                  4113

<210> 12
<211> 1370
<212> PRT
<213> Homo sapiens

<220>
<221> SOURCE
<222> 1..1370
<223> /mol_type="protein"
      /note="insulin receptor"
      /organism="Homo sapiens"

<400> 12

<210> 13
<211> 5769
<212> DNA
<213> artificial sequences

<220>
<221> source
<222> 1..5769
<223> /mol_type="DNA"
      /note="human insulin receptor-luc fusion"
      /organism="artificial sequences"

<400> 13
atgggcaccg ggggccggcg gggggcggcg gccgcgccgc tgctggtggc ggtggccgcg      60

ctgctactgg gcgccgcggg ccacctgtac cccggagagg tgtgtcccgg catggatatc     120

cggaacaacc tcactaggtt gcatgagctg gagaattgct ctgtcatcga aggacacttg     180

cagatactct tgatgttcaa aacgaggccc gaagatttcc gagacctcag tttccccaaa     240

ctcatcatga tcactgatta cttgctgctc ttccgggtct atgggctcga gagcctgaag     300

gacctgttcc ccaacctcac ggtcatccgg ggatcacgac tgttctttaa ctacgcgctg     360

gtcatcttcg agatggttca cctcaaggaa ctcggcctct acaacctgat gaacatcacc     420

cggggttctg tccgcatcga gaagaacaat gagctctgtt acttggccac tatcgactgg     480

tcccgtatcc tggattccgt ggaggataat tacatcgtgt tgaacaaaga tgacaacgag     540

gagtgtggag acatctgtcc gggtaccgcg aagggcaaga ccaactgccc cgccaccgtc     600

atcaacgggc agtttgtcga acgatgttgg actcatagtc actgccagaa agtttgcccg     660

accatctgta agtcacacgg ctgcaccgcc gaaggcctct gttgccacag cgagtgcctg     720

ggcaactgtt ctcagcccga cgaccccacc aagtgcgtgg cctgccgcaa cttctacctg     780

gacggcaggt gtgtggagac ctgcccgccc ccgtactacc acttccagga ctggcgctgt     840

gtgaacttca gcttctgcca ggacctgcac cacaaatgca agaactcgcg gaggcagggc     900

tgccaccagt acgtcattca caacaacaag tgcatccctg agtgtccctc cgggtacacg     960

atgaattcca gcaacttgct gtgcacccca tgcctgggtc cctgtcccaa ggtgtgccac    1020

ctcctagaag gcgagaagac catcgactcg gtgacgtctg cccaggagct ccgaggatgc    1080

accgtcatca acgggagtct gatcatcaac attcgaggag gcaacaatct ggcagctgag    1140

ctagaagcca acctcggcct cattgaagaa atttcagggt atctaaaaat ccgccgatcc    1200

tacgctctgg tgtcactttc cttcttccgg aagttacgtc tgattcgagg agagaccttg    1260

gaaattggga actactcctt ctatgccttg gacaaccaga acctaaggca gctctgggac    1320

tggagcaaac acaacctcac catcactcag gggaaactct tcttccacta taaccccaaa    1380

ctctgcttgt cagaaatcca caagatggaa gaagtttcag gaaccaaggg gcgccaggag    1440

agaaacgaca ttgccctgaa gaccaatggg gaccaggcat cctgtgaaaa tgagttactt    1500

aaattttctt acattcggac atcttttgac aagatcttgc tgagatggga gccgtactgg 1560

ccccccgact tccgagacct cttggggttc atgctgttct acaaagaggc cccttatcag 1620

aatgtgacgg agttcgacgg gcaggatgcg tgtggttcca acagttggac ggtggtagac 1680

attgacccac ccctgaggtc caacgacccc aaatcacaga accacccagg gtggctgatg 1740

cggggtctca agccctggac ccagtatgcc atctttgtga agaccctggt caccttttcg 1800

gatgaacgcc ggacctatgg ggccaagagt gacatcattt atgtccagac agatgccacc 1860

aacccctctg tgcccctgga tccaatctca gtgtctaact catcatccca gattattctg 1920

aagtggaaac caccctccga ccccaatggc aacatcaccc actacctggt tttctgggag 1980

aggcaggcgg aagacagtga gctgttcgag ctggattatt gcctcaaagg gctgaagctg 2040

ccctcgagga cctggtctcc accattcgag tctgaagatt ctcagaagca caaccagagt 2100

gagtatgagg attcggccgg cgaatgctgc tcctgtccaa agacagactc tcagatcctg 2160

aaggagctgg aggagtcctc gtttaggaag acgtttgagg attacctgca caacgtggtt 2220

ttcgtcccca ggccatctcg gaaacgcagg tcccttggcg atgttgggaa tgtgacggtg 2280

gccgtgccca cggtggcagc tttccccaac acttcctcga ccagcgtgcc cacgagtccg 2340

gaggagcaca ggccttttga gaaggtggtg aacaaggagt cgctggtcat ctccggcttg 2400

cgacacttca cgggctatcg catcgagctg caggcttgca accaggacac ccctgaggaa 2460

cggtgcagtg tggcagccta cgtcagtgcg aggaccatgc ctgaagccaa ggctgatgac 2520

attgttggcc ctgtgacgca tgaaatcttt gagaacaacg tcgtccactt gatgtggcag 2580

gagccgaagg agcccaatgg tctgatcgtg ctgtatgaag tgagttatcg gcgatatggt 2640

gatgaggagc tgcatctctg cgtctcccgc aagcacttcg ctctggaacg gggctgcagg 2700

ctgcgtgggc tgtcaccggg gaactacagc gtgcgaatcc gggccacctc ccttgcgggc 2760

aacggctctt ggacggaacc cacctatttc tacgtgacag actatttaga cgtcccgtca 2820

aatattgcaa aaattatcat cggccccctc atctttgtct ttctcttcag tgttgtgatt 2880

ggaagtattt atctattcct gagaaagagg cagccagatg ggccgctggg accgctttac 2940

gcttcttcaa accctgagta tctcagtgcc agtgatgtgt ttccatgctc tgtgtacgtg 3000

ccggacgagt gggaggtgtc tcgagagaag atcaccctcc ttcgagagct ggggcagggc 3060

tccttcggca tggtgtatga gggcaatgcc agggacatca tcaagggtga ggcagagacc 3120

cgcgtggcgg tgaagacggt caacgagtca gccagtctcc gagagcggat tgagttcctc 3180

aatgaggcct cggtcatgaa gggcttcacc tgccatcatg tggtgcgcct cctgggagtg 3240

gtgtccaagg gccagcccac gctggtggtg atggagctga tggctcacgg agacctgaag 3300

agctacctcc gttctctgcg gccagaggct gagaataatc ctggccgccc tccccctacc 3360

cttcaagaga tgattcagat ggcggcagag attgctgacg ggatggccta cctgaacgcc 3420

aagaagtttg tgcatcggga cctggcagcg agaaactgca tggtcgccca tgattttact 3480

gtcaaaattg gagactttgg aatgaccaga gacatctatg aaacggatta ctaccggaaa 3540

gggggcaagg gtctgctccc tgtacggtgg atggcaccgg agtccctgaa ggatggggtc 3600

ttcaccactt cttctgacat gtggtccttt ggcgtggtcc tttgggaaat caccagcttg 3660

gcagaacagc cttaccaagg cctgtctaat gaacaggtgt tgaaatttgt catggatgga 3720

gggtatctgg atcaacccga caactgtcca gagagagtca ctgacctcat gcgcatgtgc 3780

tggcaattca accccaagat gaggccaacc ttcctggaga ttgtcaacct gctcaaggac 3840

gacctgcacc ccagctttcc agaggtgtcg ttcttccaca gcgaggagaa caaggctccc 3900

gagagtgagg agctggagat ggagtttgag gacatggaga atgtgcccct ggaccgttcc 3960

tcgcactgtc agaggaggga ggcgggggc cgggatggag ggtcctcgct gggtttcaag 4020

cggagctacg aggaacacat cccttacaca cacatgaacg gaggcaagaa aaacgggcgg 4080

attctgacct tgcctcggtc caatccttcc caattggtca ccgacgccaa aaacataaag 4140

aaaggcccgg cgccattcta tccgctggaa gatggaaccg ctggagagca actgcataag 4200

gctatgaaga gatacgccct ggttcctgga acaattgctt ttacagatgc acatatcgag 4260

gtggacatca cttacgctga gtacttcgaa atgtccgttc ggttggcaga agctatgaaa 4320

cgatatgggc tgaatacaaa tcacagaatc gtcgtatgca gtgaaaactc tcttcaattc 4380

tttatgccgg tgttgggcgc gttatttatc ggagttgcag ttgcgcccgc gaacgacatt 4440

tataatgaac gtgaattgct caacagtatg ggcatttcgc agcctaccgt ggtgttcgtt 4500

tccaaaaagg ggttgcaaaa aattttgaac gtgcaaaaaa agctcccaat catccaaaaa 4560

attattatca tggattctaa aacggattac cagggatttc agtcgatgta cacgttcgtc 4620

acatctcatc tacctcccgg ttttaatgaa tacgattttg tgccagagtc cttcgatagg 4680

gacaagacaa ttgcactgat catgaactcc tctggatcta ctggtctgcc taaaggtgtc 4740

gctctgcctc atagaactgc ctgcgtgaga ttctcgcatg ccagagatcc tatttttggc 4800

aatcaaatca ttccggatac tgcgatttta agtgttgttc cattccatca cggttttgga 4860

atgtttacta cactcggata tttgatatgt ggatttcgag tcgtcttaat gtatagattt 4920

gaagaagagc tgtttctgag gagccttcag gattacaaga ttcaaagtgc gctgctggtg 4980

ccaaccctat tctccttctt cgccaaaagc actctgattg acaaatacga tttatctaat 5040

ttacacgaaa ttgcttctgg tggcgctccc ctctctaagg aagtcgggga agcggttgcc 5100

aagaggttcc atctgccagg tatcaggcaa ggatatgggc tcactgagac tacatcagct 5160

attctgatta cacccgaggg ggatgataaa ccgggcgcgg tcggtaaagt tgttccattt 5220

tttgaagcga aggttgtgga tctggatacc gggaaaacgc tgggcgttaa tcaaagaggc 5280

gaactgtgtg tgagaggtcc tatgattatg tccggttatg taaacaatcc ggaagcgacc 5340

aacgccttga ttgacaagga tggatggcta cattctggag acatagctta ctgggacgaa 5400

gacgaacact tcttcatcgt tgaccgcctg aagtctctga ttaagtacaa aggctatcag 5460

gtggctcccg ctgaattgga atccatcttg ctccaacacc ccaacatctt cgacgcaggt 5520

gtcgcaggtc ttcccgacga tgacgccggt gaacttcccg ccgccgttgt tgttttggag 5580

cacggaaaga cgatgacgga aaaagagatc gtggattacg tcgccagtca agtaacaacc 5640

gcgaaaaagt tgcgcggagg agttgtgttt gtggacgaag taccgaaagg tcttaccgga 5700

aaactcgacg caagaaaaat cagagagatc ctcataaagg ccaagaaggg cggaaagatc 5760

gccgtgtaa 5769

<210> 14
<211> 1922
<212> PRT
<213> artificial sequences

<220>
<221> SOURCE
<222> 1..1922
<223> /mol_type="protein"
/note="hIR-luc fusion protein"
/organism="artificial sequences"

<400> 14
Met Gly Thr Gly Gly Arg Arg Gly Ala Ala Ala Ala Pro Leu Leu Val
1               5                   10                  15
Ala Val Ala Ala Leu Leu Leu Gly Ala Ala Gly His Leu Tyr Pro Gly
            20                  25                  30
Glu Val Cys Pro Gly Met Asp Ile Arg Asn Asn Leu Thr Arg Leu His
        35                  40                  45
Glu Leu Glu Asn Cys Ser Val Ile Glu Gly His Leu Gln Ile Leu Leu
    50                  55                  60
Met Phe Lys Thr Arg Pro Glu Asp Phe Arg Asp Leu Ser Phe Pro Lys
65                  70                  75                  80
Leu Ile Met Ile Thr Asp Tyr Leu Leu Leu Phe Arg Val Tyr Gly Leu
                85                  90                  95
Glu Ser Leu Lys Asp Leu Phe Pro Asn Leu Thr Val Ile Arg Gly Ser
            100                 105                 110
Arg Leu Phe Phe Asn Tyr Ala Leu Val Ile Phe Glu Met Val His Leu
        115                 120                 125
Lys Glu Leu Gly Leu Tyr Asn Leu Met Asn Ile Thr Arg Gly Ser Val
    130                 135                 140
Arg Ile Glu Lys Asn Asn Glu Leu Cys Tyr Leu Ala Thr Ile Asp Trp
145                 150                 155                 160
Ser Arg Ile Leu Asp Ser Val Glu Asp Asn Tyr Ile Val Leu Asn Lys
                165                 170                 175
Asp Asp Asn Glu Glu Cys Gly Asp Ile Cys Pro Gly Thr Ala Lys Gly
            180                 185                 190
Lys Thr Asn Cys Pro Ala Thr Val Ile Asn Gly Gln Phe Val Glu Arg
        195                 200                 205
Cys Trp Thr His Ser His Cys Gln Lys Val Cys Pro Thr Ile Cys Lys
    210                 215                 220
Ser His Gly Cys Thr Ala Glu Gly Leu Cys Cys His Ser Glu Cys Leu
225                 230                 235                 240
Gly Asn Cys Ser Gln Pro Asp Asp Pro Thr Lys Cys Val Ala Cys Arg
                245                 250                 255
Asn Phe Tyr Leu Asp Gly Arg Cys Val Glu Thr Cys Pro Pro Pro Tyr
            260                 265                 270
Tyr His Phe Gln Asp Trp Arg Cys Val Asn Phe Ser Phe Cys Gln Asp
        275                 280                 285
Leu His His Lys Cys Lys Asn Ser Arg Arg Gln Gly Cys His Gln Tyr

290                 295                 300
Val Ile His Asn Asn Lys Cys Ile Pro Glu Cys Pro Ser Gly Tyr Thr
305                 310                 315                 320
Met Asn Ser Ser Asn Leu Leu Cys Thr Pro Cys Leu Gly Pro Cys Pro
                325                 330                 335
Lys Val Cys His Leu Leu Glu Gly Glu Lys Thr Ile Asp Ser Val Thr
            340                 345                 350
Ser Ala Gln Glu Leu Arg Gly Cys Thr Val Ile Asn Gly Ser Leu Ile
        355                 360                 365
Ile Asn Ile Arg Gly Gly Asn Asn Leu Ala Ala Glu Leu Glu Ala Asn
    370                 375                 380
Leu Gly Leu Ile Glu Glu Ile Ser Gly Tyr Leu Lys Ile Arg Arg Ser
385                 390                 395                 400
Tyr Ala Leu Val Ser Leu Ser Phe Phe Arg Lys Leu Arg Leu Ile Arg
                405                 410                 415
Gly Glu Thr Leu Glu Ile Gly Asn Tyr Ser Phe Tyr Ala Leu Asp Asn
            420                 425                 430
Gln Asn Leu Arg Gln Leu Trp Asp Trp Ser Lys His Asn Leu Thr Ile
        435                 440                 445
Thr Gln Gly Lys Leu Phe Phe His Tyr Asn Pro Lys Leu Cys Leu Ser
    450                 455                 460
Glu Ile His Lys Met Glu Glu Val Ser Gly Thr Lys Gly Arg Gln Glu
465                 470                 475                 480
Arg Asn Asp Ile Ala Leu Lys Thr Asn Gly Asp Gln Ala Ser Cys Glu
                485                 490                 495
Asn Glu Leu Leu Lys Phe Ser Tyr Ile Arg Thr Ser Phe Asp Lys Ile
            500                 505                 510
Leu Leu Arg Trp Glu Pro Tyr Trp Pro Pro Asp Phe Arg Asp Leu Leu
        515                 520                 525
Gly Phe Met Leu Phe Tyr Lys Glu Ala Pro Tyr Gln Asn Val Thr Glu
    530                 535                 540
Phe Asp Gly Gln Asp Ala Cys Gly Ser Asn Ser Trp Thr Val Val Asp
545                 550                 555                 560
Ile Asp Pro Pro Leu Arg Ser Asn Asp Pro Lys Ser Gln Asn His Pro
                565                 570                 575
Gly Trp Leu Met Arg Gly Leu Lys Pro Trp Thr Gln Tyr Ala Ile Phe
            580                 585                 590
Val Lys Thr Leu Val Thr Phe Ser Asp Glu Arg Arg Thr Tyr Gly Ala
        595                 600                 605
Lys Ser Asp Ile Ile Tyr Val Gln Thr Asp Ala Thr Asn Pro Ser Val
    610                 615                 620
Pro Leu Asp Pro Ile Ser Val Ser Asn Ser Ser Ser Gln Ile Ile Leu
625                 630                 635                 640
Lys Trp Lys Pro Pro Ser Asp Pro Asn Gly Asn Ile Thr His Tyr Leu
                645                 650                 655
Val Phe Trp Glu Arg Gln Ala Glu Asp Ser Glu Leu Phe Glu Leu Asp
            660                 665                 670
Tyr Cys Leu Lys Gly Leu Lys Leu Pro Ser Arg Thr Trp Ser Pro Pro
        675                 680                 685
Phe Glu Ser Glu Asp Ser Gln Lys His Asn Gln Ser Glu Tyr Glu Asp
    690                 695                 700
Ser Ala Gly Glu Cys Cys Ser Cys Pro Lys Thr Asp Ser Gln Ile Leu
705                 710                 715                 720
Lys Glu Leu Glu Glu Ser Ser Phe Arg Lys Thr Phe Glu Asp Tyr Leu
                725                 730                 735
His Asn Val Val Phe Val Pro Arg Pro Ser Arg Lys Arg Arg Ser Leu
            740                 745                 750
Gly Asp Val Gly Asn Val Thr Val Ala Val Pro Thr Val Ala Ala Phe
        755                 760                 765
Pro Asn Thr Ser Ser Thr Ser Val Pro Thr Ser Pro Glu Glu His Arg
    770                 775                 780
Pro Phe Glu Lys Val Val Asn Lys Glu Ser Leu Val Ile Ser Gly Leu
785                 790                 795                 800
Arg His Phe Thr Gly Tyr Arg Ile Glu Leu Gln Ala Cys Asn Gln Asp
                805                 810                 815

Thr Pro Glu Glu Arg Cys Ser Val Ala Ala Tyr Val Ser Ala Arg Thr
            820                 825                 830
Met Pro Glu Ala Lys Ala Asp Asp Ile Val Gly Pro Val Thr His Glu
        835                 840                 845
Ile Phe Glu Asn Asn Val Val His Leu Met Trp Gln Glu Pro Lys Glu
    850                 855                 860
Pro Asn Gly Leu Ile Val Leu Tyr Glu Val Ser Tyr Arg Arg Tyr Gly
865                 870                 875                 880
Asp Glu Glu Leu His Leu Cys Val Ser Arg Lys His Phe Ala Leu Glu
                885                 890                 895
Arg Gly Cys Arg Leu Arg Gly Leu Ser Pro Gly Asn Tyr Ser Val Arg
            900                 905                 910
Ile Arg Ala Thr Ser Leu Ala Gly Asn Gly Ser Trp Thr Glu Pro Thr
        915                 920                 925
Tyr Phe Tyr Val Thr Asp Tyr Leu Asp Val Pro Ser Asn Ile Ala Lys
    930                 935                 940
Ile Ile Ile Gly Pro Leu Ile Phe Val Phe Leu Phe Ser Val Val Ile
945                 950                 955                 960
Gly Ser Ile Tyr Leu Phe Leu Arg Lys Arg Gln Pro Asp Gly Pro Leu
                965                 970                 975
Gly Pro Leu Tyr Ala Ser Ser Asn Pro Glu Tyr Leu Ser Ala Ser Asp
            980                 985                 990
Val Phe Pro Cys Ser Val Tyr Val Pro Asp Glu Trp Glu Val Ser Arg
        995                 1000                1005
Glu Lys Ile Thr Leu Leu Arg Glu Leu Gly Gln Gly Ser Phe Gly Met
    1010                1015                1020
Val Tyr Glu Gly Asn Ala Arg Asp Ile Ile Lys Gly Glu Ala Glu Thr
1025                1030                1035                1040
Arg Val Ala Val Lys Thr Val Asn Glu Ser Ala Ser Leu Arg Glu Arg
                1045                1050                1055
Ile Glu Phe Leu Asn Glu Ala Ser Val Met Lys Gly Phe Thr Cys His
            1060                1065                1070
His Val Val Arg Leu Leu Gly Val Val Ser Lys Gly Gln Pro Thr Leu
        1075                1080                1085
Val Val Met Glu Leu Met Ala His Gly Asp Leu Lys Ser Tyr Leu Arg
    1090                1095                1100
Ser Leu Arg Pro Glu Ala Glu Asn Asn Pro Gly Arg Pro Pro Pro Thr
1105                1110                1115                1120
Leu Gln Glu Met Ile Gln Met Ala Ala Glu Ile Ala Asp Gly Met Ala
                1125                1130                1135
Tyr Leu Asn Ala Lys Lys Phe Val His Arg Asp Leu Ala Ala Arg Asn
            1140                1145                1150
Cys Met Val Ala His Asp Phe Thr Val Lys Ile Gly Asp Phe Gly Met
        1155                1160                1165
Thr Arg Asp Ile Tyr Glu Thr Asp Tyr Tyr Arg Lys Gly Gly Lys Gly
    1170                1175                1180
Leu Leu Pro Val Arg Trp Met Ala Pro Glu Ser Leu Lys Asp Gly Val
1185                1190                1195                1200
Phe Thr Thr Ser Ser Asp Met Trp Ser Phe Gly Val Val Leu Trp Glu
                1205                1210                1215
Ile Thr Ser Leu Ala Glu Gln Pro Tyr Gln Gly Leu Ser Asn Glu Gln
            1220                1225                1230
Val Leu Lys Phe Val Met Asp Gly Gly Tyr Leu Asp Gln Pro Asp Asn
        1235                1240                1245
Cys Pro Glu Arg Val Thr Asp Leu Met Arg Met Cys Trp Gln Phe Asn
    1250                1255                1260
Pro Lys Met Arg Pro Thr Phe Leu Glu Ile Val Asn Leu Leu Lys Asp
1265                1270                1275                1280
Asp Leu His Pro Ser Phe Pro Glu Val Ser Phe Phe His Ser Glu Glu
                1285                1290                1295
Asn Lys Ala Pro Glu Ser Glu Glu Leu Glu Met Glu Phe Glu Asp Met
            1300                1305                1310
Glu Asn Val Pro Leu Asp Arg Ser Ser His Cys Gln Arg Glu Glu Ala
        1315                1320                1325
Gly Gly Arg Asp Gly Gly Ser Ser Leu Gly Phe Lys Arg Ser Tyr Glu

1330                1335                1340
Glu His Ile Pro Tyr Thr His Met Asn Gly Gly Lys Lys Asn Gly Arg
1345                1350                1355                1360
Ile Leu Thr Leu Pro Arg Ser Asn Pro Ser Gln Leu Val Thr Asp Ala
                1365                1370                1375
Lys Asn Ile Lys Lys Gly Pro Ala Pro Phe Tyr Pro Leu Glu Asp Gly
            1380                1385                1390
Thr Ala Gly Glu Gln Leu His Lys Ala Met Lys Arg Tyr Ala Leu Val
        1395                1400                1405
Pro Gly Thr Ile Ala Phe Thr Asp Ala His Ile Glu Val Asp Ile Thr
    1410                1415                1420
Tyr Ala Glu Tyr Phe Glu Met Ser Val Arg Leu Ala Glu Ala Met Lys
1425                1430                1435                1440
Arg Tyr Gly Leu Asn Thr Asn His Arg Ile Val Val Cys Ser Glu Asn
                1445                1450                1455
Ser Leu Gln Phe Phe Met Pro Val Leu Gly Ala Leu Phe Ile Gly Val
            1460                1465                1470
Ala Val Ala Pro Ala Asn Asp Ile Tyr Asn Glu Arg Glu Leu Leu Asn
        1475                1480                1485
Ser Met Gly Ile Ser Gln Pro Thr Val Val Phe Val Ser Lys Lys Gly
    1490                1495                1500
Leu Gln Lys Ile Leu Asn Val Gln Lys Lys Leu Pro Ile Ile Gln Lys
1505                1510                1515                1520
Ile Ile Ile Met Asp Ser Lys Thr Asp Tyr Gln Gly Phe Gln Ser Met
                1525                1530                1535
Tyr Thr Phe Val Thr Ser His Leu Pro Pro Gly Phe Asn Glu Tyr Asp
            1540                1545                1550
Phe Val Pro Glu Ser Phe Asp Arg Asp Lys Thr Ile Ala Leu Ile Met
        1555                1560                1565
Asn Ser Ser Gly Ser Thr Gly Leu Pro Lys Gly Val Ala Leu Pro His
    1570                1575                1580
Arg Thr Ala Cys Val Arg Phe Ser His Ala Arg Asp Pro Ile Phe Gly
1585                1590                1595                1600
Asn Gln Ile Ile Pro Asp Thr Ala Ile Leu Ser Val Val Pro Phe His
                1605                1610                1615
His Gly Phe Gly Met Phe Thr Thr Leu Gly Tyr Leu Ile Cys Gly Phe
            1620                1625                1630
Arg Val Val Leu Met Tyr Arg Phe Glu Glu Glu Leu Phe Leu Arg Ser
        1635                1640                1645
Leu Gln Asp Tyr Lys Ile Gln Ser Ala Leu Leu Val Pro Thr Leu Phe
    1650                1655                1660
Ser Phe Phe Ala Lys Ser Thr Leu Ile Asp Lys Tyr Asp Leu Ser Asn
1665                1670                1675                1680
Leu His Glu Ile Ala Ser Gly Gly Ala Pro Leu Ser Lys Glu Val Gly
                1685                1690                1695
Glu Ala Val Ala Lys Arg Phe His Leu Pro Gly Ile Arg Gln Gly Tyr
            1700                1705                1710
Gly Leu Thr Glu Thr Thr Ser Ala Ile Leu Ile Thr Pro Glu Gly Asp
        1715                1720                1725
Asp Lys Pro Gly Ala Val Gly Lys Val Val Pro Phe Phe Glu Ala Lys
    1730                1735                1740
Val Val Asp Leu Asp Thr Gly Lys Thr Leu Gly Val Asn Gln Arg Gly
1745                1750                1755                1760
Glu Leu Cys Val Arg Gly Pro Met Ile Met Ser Gly Tyr Val Asn Asn
                1765                1770                1775
Pro Glu Ala Thr Asn Ala Leu Ile Asp Lys Asp Gly Trp Leu His Ser
            1780                1785                1790
Gly Asp Ile Ala Tyr Trp Asp Glu Asp Glu His Phe Phe Ile Val Asp
        1795                1800                1805
Arg Leu Lys Ser Leu Ile Lys Tyr Lys Gly Tyr Gln Val Ala Pro Ala
    1810                1815                1820
Glu Leu Glu Ser Ile Leu Leu Gln His Pro Asn Ile Phe Asp Ala Gly
1825                1830                1835                1840
Val Ala Gly Leu Pro Asp Asp Asp Ala Gly Glu Leu Pro Ala Ala Val
                1845                1850                1855

Val Val Leu Glu His Gly Lys Thr Met Thr Glu Lys Glu Ile Val Asp
        1860                1865                1870
Tyr Val Ala Ser Gln Val Thr Thr Ala Lys Lys Leu Arg Gly Gly Val
    1875                1880                1885
Val Phe Val Asp Glu Val Pro Lys Gly Leu Thr Gly Lys Leu Asp Ala
1890                1895                1900
Arg Lys Ile Arg Glu Ile Leu Ile Lys Ala Lys Lys Gly Gly Lys Ile
1905                1910                1915                1920
Ala Val

<210> 15
<211> 2781
<212> DNA
<213> artificial sequences

<220>
<221> source
<222> 1..2781
<223> /mol_type="DNA"
      /note="extracellular domain of human IGF1R"
      /organism="artificial sequences"

<400> 15
atgaagtctg gctccggagg agggtccccg acctcgctgt gggggctcct gtttctctcc      60

gccgcgctct cgctctggcc gacgagtgga gaaatctgcg ggccaggcat cgacatccgc     120

aacgactatc agcagctgaa gcgcctggag aactgcacgg tgatcgaggg ctacctccac     180

atcctgctca tctccaaggc cgaggactac cgcagctacc gcttccccaa gctcacggtc     240

attaccgagt acttgctgct gttccgagtg gctggcctcg agagcctcgg agacctcttc     300

cccaacctca cggtcatccg cggctggaaa ctcttctaca actacgccct ggtcatcttc     360

gagatgacca atctcaagga tattgggctt tacaacctga ggaacattac tcggggggcc     420

atcaggattg agaaaaatgc tgacctctgt tacctctcca ctgtggactg gtccctgatc     480

ctggatgcgg tgtccaataa ctacattgtg gggaataagc ccccaaagga atgtggggac     540

ctgtgtccag ggaccatgga ggagaagccg atgtgtgaga agaccaccat caacaatgag     600

tacaactacc gctgctggac cacaaaccgc tgccagaaaa tgtgcccaag cacgtgtggg     660

aagcgggcgt gcaccgagaa caatgagtgc tgccaccccg agtgcctggg cagctgcagc     720

gcgcctgaca acgacacggc ctgtgtagct tgccgccact actactatgc cggtgtctgt     780

gtgcctgcct gcccgcccaa cacctacagg tttgagggct ggcgctgtgt ggaccgtgac     840

ttctgcgcca acatcctcag cgccgagagc agcgactccg aggggtttgt gatccacgac     900

ggcgagtgca tgcaggagtg cccctcgggc ttcatccgca acggcagcca gagcatgtac     960

tgcatccctt gtgaaggtcc ttgcccgaag gtctgtgagg aagaaaagaa aacaaagacc    1020

attgattctg ttacttctgc tcagatgctc caaggatgca ccatcttcaa gggcaatttg    1080

ctcattaaca tccgacgggg gaataacatt gcttcagagc tggagaactt catggggctc    1140

atcgaggtgg tgacgggcta cgtgaagatc cgccattctc atgccttggt ctccttgtcc    1200

ttcctaaaaa accttcgcct catcctagga gaggagcagc tagaagggaa ttactccttc    1260

tacgtcctcg acaaccagaa cttgcagcaa ctgtgggact gggaccaccg caacctgacc 1320

atcaaagcag ggaaaatgta ctttgctttc aatcccaaat tatgtgtttc cgaaatttac 1380

cgcatggagg aagtgacggg gactaaaggg cgccaaagca aaggggacat aaacaccagg 1440

aacaacgggg agagagcctc ctgtgaaagt gacgtcctgc atttcacctc caccaccacg 1500

tcgaagaatc gcatcatcat aacctggcac cggtaccggc cccctgacta cagggatctc 1560

atcagcttca ccgtttacta caaggaagca ccctttaaga atgtcacaga gtatgatggg 1620

caggatgcct gcggctccaa cagctggaac atggtggacg tggacctccc gcccaacaag 1680

gacgtggagc ccggcatctt actacatggg ctgaagccct ggactcagta cgccgtttac 1740

gtcaaggctg tgaccctcac catggtggag aacgaccata tccgtggggc caagagtgag 1800

atcttgtaca ttcgcaccaa tgcttcagtt ccttccattc ccttggacgt tctttcagca 1860

tcgaactcct cttctcagtt aatcgtgaag tggaaccctc cctctctgcc caacggcaac 1920

ctgagttact acattgtgcg ctggcagcgg cagcctcagg acggctacct ttaccggcac 1980

aattactgct ccaaagacaa aatccccatc aggaagtatg ccgacggcac catcgacatt 2040

gaggaggtca cagagaaccc caagactgag gtgtgtggtg gggagaaagg gccttgctgc 2100

gcctgcccca aaactgaagc cgagaagcag gccgagaagg aggaggctga ataccgcaaa 2160

gtctttgaga atttcctgca caactccatc ttcgtgccca gacctgaaag gaagcggaga 2220

gatgtcatgc aagtggccaa caccaccatg tccagccgaa gcaggaacac cacggccgca 2280

gacacctaca acatcaccga cccggaagag ctggagacag agtacccttt ctttgagagc 2340

agagtggata acaaggagag aactgtcatt tctaaccttc ggcctttcac attgtaccgc 2400

atcgatatcc acagctgcaa ccacgaggct gagaagctgg gctgcagcgc ctccaacttc 2460

gtctttgcaa ggactatgcc cgcagaagga gcagatgaca ttcctgggcc agtgacctgg 2520

gagccaaggc ctgaaaactc catcttttta aagtggccgg aacctgagaa tcccaatgga 2580

ttgattctaa tgtatgaaat aaaatacgga tcacaagttg aggatcagcg agaatgtgtg 2640

tccagacagg aatacaggaa gtatggaggg gccaagctaa accggctaaa cccggggaac 2700

tacacagccc ggattcaggc cacatctctc tctgggaatg ggtcgtggac agatcctgtg 2760

ttcttctatg tccaggccaa a 2781

<210> 16
<211> 927
<212> PRT
<213> artificial sequences

<220>
<221> SOURCE
<222> 1..927
<223> /mol_type="protein"
/note="extracellular domain of human IGF1R"
/organism="artificial sequences"

<400> 16
Met Lys Ser Gly Ser Gly Gly Gly Ser Pro Thr Ser Leu Trp Gly Leu
1               5                   10                  15
Leu Phe Leu Ser Ala Ala Leu Ser Leu Trp Pro Thr Ser Gly Glu Ile
            20                  25                  30
Cys Gly Pro Gly Ile Asp Ile Arg Asn Asp Tyr Gln Gln Leu Lys Arg
        35                  40                  45
Leu Glu Asn Cys Thr Val Ile Glu Gly Tyr Leu His Ile Leu Leu Ile
    50                  55                  60
Ser Lys Ala Glu Asp Tyr Arg Ser Tyr Arg Phe Pro Lys Leu Thr Val
65                  70                  75                  80
Ile Thr Glu Tyr Leu Leu Leu Phe Arg Val Ala Gly Leu Glu Ser Leu
                85                  90                  95
Gly Asp Leu Phe Pro Asn Leu Thr Val Ile Arg Gly Trp Lys Leu Phe
            100                 105                 110
Tyr Asn Tyr Ala Leu Val Ile Phe Glu Met Thr Asn Leu Lys Asp Ile
        115                 120                 125
Gly Leu Tyr Asn Leu Arg Asn Ile Thr Arg Gly Ala Ile Arg Ile Glu
    130                 135                 140
Lys Asn Ala Asp Leu Cys Tyr Leu Ser Thr Val Asp Trp Ser Leu Ile
145                 150                 155                 160
Leu Asp Ala Val Ser Asn Asn Tyr Ile Val Gly Asn Lys Pro Pro Lys
                165                 170                 175
Glu Cys Gly Asp Leu Cys Pro Gly Thr Met Glu Glu Lys Pro Met Cys
            180                 185                 190
Glu Lys Thr Thr Ile Asn Asn Glu Tyr Asn Tyr Arg Cys Trp Thr Thr
        195                 200                 205
Asn Arg Cys Gln Lys Met Cys Pro Ser Thr Cys Gly Lys Arg Ala Cys
    210                 215                 220
Thr Glu Asn Asn Glu Cys Cys His Pro Glu Cys Leu Gly Ser Cys Ser
225                 230                 235                 240
Ala Pro Asp Asn Asp Thr Ala Cys Val Ala Cys Arg His Tyr Tyr Tyr
                245                 250                 255
Ala Gly Val Cys Val Pro Ala Cys Pro Pro Asn Thr Tyr Arg Phe Glu
            260                 265                 270
Gly Trp Arg Cys Val Asp Arg Asp Phe Cys Ala Asn Ile Leu Ser Ala
        275                 280                 285
Glu Ser Ser Asp Ser Glu Gly Phe Val Ile His Asp Gly Glu Cys Met
    290                 295                 300
Gln Glu Cys Pro Ser Gly Phe Ile Arg Asn Gly Ser Gln Ser Met Tyr
305                 310                 315                 320
Cys Ile Pro Cys Glu Gly Pro Cys Pro Lys Val Cys Glu Glu Glu Lys
                325                 330                 335
Lys Thr Lys Thr Ile Asp Ser Val Thr Ser Ala Gln Met Leu Gln Gly
            340                 345                 350
Cys Thr Ile Phe Lys Gly Asn Leu Leu Ile Asn Ile Arg Arg Gly Asn
        355                 360                 365
Asn Ile Ala Ser Glu Leu Glu Asn Phe Met Gly Leu Ile Glu Val Val
    370                 375                 380
Thr Gly Tyr Val Lys Ile Arg His Ser His Ala Leu Val Ser Leu Ser
385                 390                 395                 400
Phe Leu Lys Asn Leu Arg Leu Ile Leu Gly Glu Glu Gln Leu Glu Gly
                405                 410                 415
Asn Tyr Ser Phe Tyr Val Leu Asp Asn Gln Asn Leu Gln Gln Leu Trp
            420                 425                 430
Asp Trp Asp His Arg Asn Leu Thr Ile Lys Ala Gly Lys Met Tyr Phe
        435                 440                 445
Ala Phe Asn Pro Lys Leu Cys Val Ser Glu Ile Tyr Arg Met Glu Glu
    450                 455                 460
Val Thr Gly Thr Lys Gly Arg Gln Ser Lys Gly Asp Ile Asn Thr Arg
465                 470                 475                 480
Asn Asn Gly Glu Arg Ala Ser Cys Glu Ser Asp Val Leu His Phe Thr
                485                 490                 495
Ser Thr Thr Thr Ser Lys Asn Arg Ile Ile Ile Thr Trp His Arg Tyr

500                 505                 510
Arg Pro Pro Asp Tyr Arg Asp Leu Ile Ser Phe Thr Val Tyr Tyr Lys
        515                 520                 525
Glu Ala Pro Phe Lys Asn Val Thr Glu Tyr Asp Gly Gln Asp Ala Cys
    530                 535                 540
Gly Ser Asn Ser Trp Asn Met Val Asp Val Asp Leu Pro Pro Asn Lys
545                 550                 555                 560
Asp Val Glu Pro Gly Ile Leu Leu His Gly Leu Lys Pro Trp Thr Gln
                565                 570                 575
Tyr Ala Val Tyr Val Lys Ala Val Thr Leu Thr Met Val Glu Asn Asp
            580                 585                 590
His Ile Arg Gly Ala Lys Ser Glu Ile Leu Tyr Ile Arg Thr Asn Ala
        595                 600                 605
Ser Val Pro Ser Ile Pro Leu Asp Val Leu Ser Ala Ser Asn Ser Ser
    610                 615                 620
Ser Gln Leu Ile Val Lys Trp Asn Pro Pro Ser Leu Pro Asn Gly Asn
625                 630                 635                 640
Leu Ser Tyr Tyr Ile Val Arg Trp Gln Arg Gln Pro Gln Asp Gly Tyr
                645                 650                 655
Leu Tyr Arg His Asn Tyr Cys Ser Lys Asp Lys Ile Pro Ile Arg Lys
            660                 665                 670
Tyr Ala Asp Gly Thr Ile Asp Ile Glu Glu Val Thr Glu Asn Pro Lys
        675                 680                 685
Thr Glu Val Cys Gly Gly Glu Lys Gly Pro Cys Cys Ala Cys Pro Lys
    690                 695                 700
Thr Glu Ala Glu Lys Gln Ala Glu Lys Glu Glu Ala Glu Tyr Arg Lys
705                 710                 715                 720
Val Phe Glu Asn Phe Leu His Asn Ser Ile Phe Val Pro Arg Pro Glu
                725                 730                 735
Arg Lys Arg Arg Asp Val Met Gln Val Ala Asn Thr Thr Met Ser Ser
            740                 745                 750
Arg Ser Arg Asn Thr Thr Ala Ala Asp Thr Tyr Asn Ile Thr Asp Pro
        755                 760                 765
Glu Glu Leu Glu Thr Glu Tyr Pro Phe Phe Glu Ser Arg Val Asp Asn
    770                 775                 780
Lys Glu Arg Thr Val Ile Ser Asn Leu Arg Pro Phe Thr Leu Tyr Arg
785                 790                 795                 800
Ile Asp Ile His Ser Cys Asn His Glu Ala Glu Lys Leu Gly Cys Ser
                805                 810                 815
Ala Ser Asn Phe Val Phe Ala Arg Thr Met Pro Ala Glu Gly Ala Asp
            820                 825                 830
Asp Ile Pro Gly Pro Val Thr Trp Glu Pro Arg Pro Glu Asn Ser Ile
        835                 840                 845
Phe Leu Lys Trp Pro Glu Pro Glu Asn Pro Asn Gly Leu Ile Leu Met
    850                 855                 860
Tyr Glu Ile Lys Tyr Gly Ser Gln Val Glu Asp Gln Arg Glu Cys Val
865                 870                 875                 880
Ser Arg Gln Glu Tyr Arg Lys Tyr Gly Gly Ala Lys Leu Asn Arg Leu
                885                 890                 895
Asn Pro Gly Asn Tyr Thr Ala Arg Ile Gln Ala Thr Ser Leu Ser Gly
            900                 905                 910
Asn Gly Ser Trp Thr Asp Pro Val Phe Phe Tyr Val Gln Ala Lys
        915                 920                 925

<210> 17
<211> 33
<212> DNA
<213> artificial sequences

<220>
<221> source
<222> 1..33
<223> /mol_type="DNA"
      /note="Primer P5"
      /organism="artificial sequences"

<400> 17
acggttaaca tgggcaccgg gggccggcgg ggg                                    33


<210> 18
<211> 33
<212> DNA
<213> artificial sequences

<220>
<221> source
<222> 1..33
<223> /mol_type="DNA"
      /note="Primer P6"
      /organism="artificial sequences"

<400> 18
acgcaattgc agcaagatct tgtcaaaaga tgt                                    33


<210> 19
<211> 36
<212> DNA
<213> artificial sequences

<220>
<221> source
<222> 1..36
<223> /mol_type="DNA"
      /note="Primer P7"
      /organism="artificial sequences"

<400> 19
atcggatcca ccatgaagtc tggctccgga ggaggg                                 36


<210> 20
<211> 83
<212> DNA
<213> artificial sequences

<220>
<221> source
<222> 1..83
<223> /mol_type="DNA"
      /note="Primer P8"
      /organism="artificial sequences"

<400> 20
atcaagcttc actttcctgc tcctggttgc tgattctctg aaagctcact gctgccaagt      60

ttggcctgga catagaagaa cac                                               83

## Description

The present invention relates to methods and kits useful in the identification of modulators of the binding properties of antibodies reactive with one or more members of the insulin receptor family and for the detection of such antibodies.

### Background of the invention

The insulin receptor family (IRF) mainly consists of four receptors that bind insulin and/or IGF1 with different affinities: insulin receptor (IR), IGF1 receptor (IGF1R), and IGF2 receptor (IGF2R), and the insulin-IGF1 hybrid receptor (IIHR). Another orphan member of this family is the insulin receptor-related receptor (IRRR).

From the above mentioned receptors, IR, IGF1R, IIHR, and IRRR belong to the family of ligand-activated tyrosine kinase receptors, whereas IGF2R is a transmembrane monomer with a large extracellular domain and no intrinsic signaling capabilities.

IR and IGF1R are expressed at the cell surface as homodimers composed of two identical monomers, or as heterodimers composed of two different receptor monomers (e.g., the insulin-IGF1 hybrid receptor (IIHR)). IIHRs are widely distributed in different mammalian tissues. They behave in a manner similar to IGF1R with respect to ligand-induced tyrosine phosphorylation signaling.

Both IGF1R and IR receptors are composed of two alpha and two beta glycosylated subunits, wherein two transmembrane alpha-beta chains form together a disulfide-linked heterotetramer (beta-alpha-alpha-beta). The receptors have an extracellular ligand binding domain, a single transmembrane domain, and a cytoplasmic domain exhibiting the tyrosine kinase activity. The extracellular domain is composed of the entire alpha subunits and a portion of the N-terminus of the beta subunits, while the intracellular portion of the beta subunits contains the tyrosine kinase (TK) domain.

While similar in structure, IGF1R and IR serve different physiological functions. IR is primarily involved in metabolic functions, whereas IGF1R mediates growth and differentiation. However, their ligands insulin and IGF-1 can induce both mitogenic and metabolic effects when binding the receptors.

Due to the structural relationship of the members of the IRF, the ligand insulin does not only bind to IR, but also to IGF1R and IIHR, the ligand IGF-1 does not only bind to IGF1R, but also to IR, IIHR, and IGF2R, and the ligand IGF-2 does not only bind to IGF2R, but also to IR, IIHR, and IGF1R.

Autoantibodies are known to play an important role in the generation of autoimmune disorders. For example, the presence of autoantibodies to IR in a subject indicates the onset of diabetes (Type B insulin resistance).

Furthermore, non-endogenous antibodies to IGF1R are currently in development to provide novel therapeutical approaches in the treatment of cancer. For example, the drug candidate's figitumumab, developed by Pfizer Inc. and dalatozumab, developed by Merck & Co Inc. are monoclonal therapeutical antibodies to IGF1R, which are currently investigated for the treatment of various types of cancer. For example, patent applications WO 2002/053596 A, WO 2004/083248 A, WO 2005/016967 A, WO 2005/016970 A, US 2005/0249730, US 2005/0084906, WO 2005/052005 A, WO 2005/058967 A, WO 2005/094376 A, WO 2006/008639 A, WO 2006/013472 A, and WO 2008/077546 A refer to the treatment of cancer by means of anti-IGF1R antibodies.

The prevalence and role of autoantibodies which are capable to bind members of the IRF is not yet fully understood and requires further investigation to successfully allow diagnosis, prevention, and treatment of autoimmune disorders (such as, e.g., insulin dependent diabetes mellitus (IDDM), Morbus Basedow, Graves Orbitopathy), obesity, neurological disorders, growth disorders, cancer generation and development (including breast, colon, ovarian, prostate, lung), and other cellular proliferative disorders and other diseases or dysfunctions. For example, dysfunctional IR signaling has been reported in disorders including diabetes type I and II, dementia, and cancer.

More specific and more sensitive assay methods may be helpful to develop a deeper understanding of these complex pathways and will serve as valuable tools in the diagnosis and research in this context, e.g., by allowing discrimination of different physiological states between a healthy individual and progressing dysfunctions. In particular, the measurement of the autoantibodies to IRF members as serological markers can be useful in the early diagnosis or differential diagnosis, in prevention and/or therapy of any of the above mentioned autoimmune disorders.

Furthermore, the improvements of present invention are enabling the identification of modulators (i.e. activators, inhibitors or otherwise affecting or triggering molecules) of the interaction of the members of the IRF and analyte antibodies that affect or trigger the receptor activity, and which therefore may be suitable as pharmaceutically effective compounds in the diagnosis, prevention and/or treatment of any of the diseases which are related to members of the IRF.

Expression of a recombinant human IGF1R-luciferase fusion protein in stably transfected HEK293 cells has been shown by W. Minich et al. (Detection of Autoantibodies to the Insulin-Like Growth Factor-1 Receptor in Patients with Graves Orbitopathy by Luminescent Immunoprecipitation Analysis. Poster Presentation ITC 2010 Paris) who showed quantification of auto-antibodies to IGF1R in sera of patients with Graves' orbitopathy using an immunoprecipitation assay (IPA).

Verch et al. (Bioanalysis September 2011, 3(18): 2107-2117) reported the development of two ELISA assays for the quantification of therapeutical anti-IGF1R antibodies in order to study their pharmacokinetic properties. These two assays are based on classical ELISA methods using labeled anti-human IgG antibodies or labeled anti-human IgGFc antibodies as the detecting reagent.

Furthermore, an ELISA Kit for human anti-insulin receptor antibody detection is provided by Uscn Life Science Inc. (Wuhan, CN). This kit is based on the principle of a sandwich enzyme immunoassay, wherein a microtiter plate has been pre-coated with the antigen. After incubation with the sample, biotin-conjugated antigen is added, and HRP-conjugated avidin is used as the detecting reagent.

Generally, it is known that the use of labeled anti-IgG- or anti-IgGFc-antibodies as the detecting reagent may form complexes with unspecifically antigen-bound antibodies, thereby resulting in false positive signals. Similarly, the biotin-avidin amplification technique is known to result in poor signal to noise ratios. Therefore, there is still the need for assay technology and performance improvement, for example, in the area of high throughput screening (HTS-) assays, where sample size is often critical and both consumption of sample volumes and assay reagents need always further reduction due to limited availabilities and the requirement of cost reduction. Moreover, epidemiological analyses or HTS-assays profit from minimal reaction step numbers, low hands-on-time during assay handling and other technical improvements.

Here, the present invention, overcomes the aforementioned problems by providing improved detection methods and kits employed in the analysis and screening of antibodies reactive with antigenic molecules which are selected from the IRF. In particular, by providing the new immunoassay methods and kits, the present invention surprisingly improves specificity and sensitivity of prior art antibody detection as discussed above. The use of the methods according to the invention is much closer to the physiological conditions and therefore allows the antigenic molecules to expose their correct three-dimensional structure. Consequently, the methods according to the present invention are not only favorable for performing automated assay formats in research and clinical laboratories but are more sensitive and specific than prior art assays.

Surprisingly, it was found by means of the methods according to the present invention that autoantibody-positive samples do cross-react with different members of the IRF. Due to the surprisingly improved sensitivity and specificity of the methods according to the present invention, the new assay format allows for a more differentiated investigation and development of novel therapeutics and diagnostic tools in the prophylaxis and treatment of disorders and diseases related to members of the IRF.

### Summary of the Invention

Therefore, it is one embodiment of the present invention to provide a method for the identification of a modulator of the binding properties of analyte antibodies reactive with one or more antigenic molecules, said method comprising: (a) providing one or more analyte antibodies reactive with one or more first and one or more second antigenic molecules; (b) providing one or more first antigenic molecules with which said analyte antibodies can interact and which first antigenic molecule is selected from the insulin receptor family (IRF); and (c) providing one or more second antigenic molecules with which analyte antibodies can interact and which second antigenic molecule is selected from the IRF; and (d) contacting said analyte antibodies as provided by step (a) and said first antigenic molecules as provided by step (b) and said second antigenic molecules as provided by step (c) simultaneously or successively with a sample to be investigated, whereby said modulator when present in said sample can interact with the said analyte antibodies and/or said antigenic molecules so as to interfere with the formation of complexes comprising [first antigenic molecule]-[analyte antibody]-[second antigenic molecule]; and (e₁) prior to, or concurrent with, or subsequent to, step (d), providing immobilizing means whereby said first antigenic molecule as present in the said complexes formed in step (d), respectively, as capable to form complexes in step (d) can be immobilized to a solid support prior to, or concurrent with, or subsequent to, step (d); and/or (e₂) prior to, or concurrent with, or subsequent to, step (d), providing second labeling means whereby said first antigenic molecule as present in the said complexes formed in step (d), respectively, as capable to form complexes in step (d) is labeled with said second labeling means prior to, or concurrent with, or subsequent to, step (d); and (f) prior to, or concurrent with, or subsequent to, step (d), providing first labeling means whereby said second antigenic molecule as present in the said complexes formed in step (d), respectively, as capable to form complexes in step (d) is labeled with said first labeling means prior to, or concurrent with, or subsequent to, step (d); and (g) prior to, or concurrent with, or subsequent to, step (d), providing one or more modulators capable to interact with the complexes formed in step (d) and/or capable to interfere with the complex formation according to step (d) and contacting said one or more modulators simultaneously or successively with the said sample, the said one or more first antigenic molecules, and/or the said one or more second antigenic molecules prior to, or concurrent with, or subsequent to step (d), or contacting said one or more modulators simultaneously or successively with the said complexes formed in or subsequent to step (d); and (h) detecting the presence of complexes formed in or subsequent to step (d).

It is another embodiment of the present invention to provide a method of detecting in a sample to be investigated the presence and/or the binding properties of analyte antibodies reactive with one or more antigenic molecules, said method comprising:
(a) providing one or more first antigenic molecules with which analyte antibodies when present in said sample can interact and which first antigenic molecule is selected from the insulin receptor family (IRF); and (b) providing one or more second antigenic molecules with which analyte antibodies when present in said sample can interact and which second antigenic molecule is selected from the IRF; and (c) contacting said first antigenic molecules as provided by step (a) and said second antigenic molecules as provided by step (b) simultaneously or successively with the sample to be investigated, whereby analyte antibodies when present in said sample can interact with said antigenic molecules so as to form complexes comprising [first antigenic molecule]-[analyte antibody]-[second antigenic molecule]; and (d₁) prior to, or concurrent with, or subsequent to, step (c), providing immobilizing means whereby said first antigenic molecule as present in the said complexes formed in step (c), respectively, as capable to form complexes in step (c) can be immobilized to a solid support prior to, or concurrent with, or subsequent to, step (c); and/or (d₂) prior to, or concurrent with, or subsequent to, step (c), providing second labeling means whereby said first antigenic molecule as present in the said complexes formed in step (c), respectively, as capable to form complexes in step (c) is labeled with said second labeling means prior to, or concurrent with, or subsequent to, step (c); and (e) prior to, or concurrent with, or subsequent to, step (c), providing first labeling means whereby said second antigenic molecule as present in the said complexes formed in step (c), respectively, as capable to form complexes in step (c) is labeled with said first labeling means prior to, or concurrent with, or subsequent to, step (c); and (g) detecting the presence of complexes formed in or subsequent to step (c) so as to provide indication of analyte antibodies present in said sample.

The methods according to the present invention are, preferably, in vitro methods. Moreover, they may comprise steps in addition to those explicitly mentioned above including sample pretreatments or evaluation of the results (e.g., with respect to the detection of the presence of complexes formed) obtained by the methods. The methods may be carried out manually and/or assisted by automation. Preferably, one or more of steps (a), (b), (c), (d), (d₁), (d₂), (e), (e₁), (e₂), (f) and/or (g) may in total or in part be assisted by automation including suitable robotic and sensory equipment for detection and/or a computer-implemented processing and/or analysis in step (g). For a person skilled in the art, it is known that the methods according to the present invention require calibration or standardization to compare the detected signals, e.g. of the presence of complexes with (one or more) known amounts of reference complex formation.

In other embodiments of the present invention the said first antigenic molecules and the said second antigenic molecules are identical or, alternatively, they are not identical (i.e. different). In other embodiments of the present invention the said first antigenic molecules and the said second antigenic molecules are not identical and belong to different members of the IRF. In other embodiments of the present invention the said first antigenic molecules and/or the said second antigenic molecules are embedded in a membrane environment. In still other embodiments the methods of the present invention allow the detection of an antibody against a member of the IRF of about 3 ng/ml, preferably of about 1 ng/ml, more preferred of about 0,3 ng/ml, even more preferred of about 0,1 ng/ml and most preferred of about 0,03 ng/ml.

In other embodiments of the methods of the present invention the one or more first antigenic molecules, which are immobilized to a solid support, are provided prior to step (c) of the method of detecting autoantibodies, respectively, prior to step (d) of the method for the identification of modulators according to the present invention.

The one or more first antigenic molecules may be immobilized to a solid support prior to contact with a sample to be investigated. Optionally, the solid support may be provided in a liquid phase (e.g., dispersion, suspension, and colloid). Such immobilized one or more first antigenic molecules are subsequently contacted with the said sample either simultaneously or successively with contact of the said sample and with one or more second antigenic molecules. The immobilized one or more first antigenic molecules when contacted with the said sample may form intermediate complexes comprising [first antigenic molecule]-[analyte antibody] wherein the one or more first antigenic molecule is immobilized to a solid support and the thus formed immobilized intermediate complex is subsequently contacted with the one or more second antigenic molecules, present in solution, so as to form the hitherto described complexes comprising [first antigenic molecule]-[analyte antibody]-[second antigenic molecule] directly or indirectly immobilized to a solid support via the first antigenic molecule.

In other embodiments of the method according to the present invention the one or more first antigenic molecules, which are provided with a labeling means, are provided prior to step (c) of the method of detecting autoantibodies, respectively, prior to step (d) of the method for the identification of modulators according to the present invention.

In another embodiment of the methods according to the present invention the one or more second antigenic molecules, which are provided with a labeling means, are provided prior to step (c) of the method of detecting autoantibodies, respectively, prior to step (d) of the method for the identification of modulators according to the present invention.

In another embodiment of the methods according to the present invention the one or more first antigenic molecules, which are immobilized to a solid support and the one or more second antigenic molecules, which are provided with a first labeling means, are both provided prior to step (c) of the method of detecting autoantibodies, respectively, prior to step (d) of the method for the identification of modulators according to the present invention.

In another embodiment of the methods according to the present invention the one or more first antigenic molecules, which are provided with a second labeling means and the one or more second antigenic molecules, which are provided with a first labeling means are both provided prior to step (c) of the method of detecting autoantibodies, respectively, prior to step (d) of the method for the identification of modulators according to the present invention.

In another embodiment of the methods according to the present invention the one or more first antigenic molecules, which are both immobilized to a solid support and provided with a second labeling means and the one or more second antigenic molecules, which are provided with a first labeling means are all provided prior to step (c) of the method of detecting autoantibodies, respectively, prior to step (d) of the method for the identification of modulators according to the present invention.

In still other embodiments of the present invention the one or more first antigenic molecules are immobilized to a solid support and the one or more second antigenic molecules are provided with labeling means, whereby the one or more second antigenic molecules are provided in solution.

In yet other embodiments of the present invention the one or more first antigenic molecules are immobilized to a solid support and the one or more second antigenic molecules are provided with labeling means, whereby the one or more first antigenic molecules and the one or more second antigenic molecules are provided in solution.

Another embodiments of the methods of the present invention comprise directly monitoring the interaction of (i) analyte antibodies present in the sample and (ii) one or more first antigenic molecules and (iii) one or more second antigenic molecules and as provided by the present invention, by employing assay techniques substantially as known in the art (e.g., non-competitive or competitive assays), for example of the sandwich type or RET type, the latter assay type not requiring immobilization and separation of the one or more first antigenic molecules from the liquid phase.

Yet another embodiment of the methods of the present invention allow for the identification of modulators, which are capable to interact with the complexes formed in step (c) and/or which are capable to interfere with the complex formation according to step (c) as defined in the method of detecting analyte antibodies according to the present invention.

Accordingly, another embodiment of the methods of detecting analyte antibodies of the present invention further comprises step (f) prior to, or concurrent with, or subsequent to, step (c), providing one or more modulators capable to interact with the complexes formed in step (c) and/or capable to interfere with the complex formation according to step (c) and contacting said one or more modulators simultaneously or successively with the said sample, the said one or more first antigenic molecules, and/or the said one or more second antigenic molecules prior to, or concurrent with, or subsequent to step (c), or contacting said one or more modulators simultaneously or successively with the said complexes formed in or subsequent to step (c).

In another embodiments of the methods according to the present invention the one or more modulators are provided prior to step (c) of the method of detecting autoantibodies, respectively, prior to step (d) of the method for the identification of modulators according to the present invention.

In still other embodiments of the methods according to the present invention one or more of the said means selected from the group consisting of the said immobilizing means, the said second labeling means, and the said first labeling means are provided prior to step (c) of the method of detecting autoantibodies, respectively, prior to step (d) of the method for the identification of modulators according to the present invention.

In a further embodiment the present invention provides a kit, which is useful for the performance of any of the methods according to the present invention comprising (a) one or more first antigenic molecules selected from the IRF as defined in one or more of the methods according to the invention; (b) one or more second antigenic molecules selected from the IRF as defined in one or more of the methods according to the invention; (c₁) immobilization means as defined in one or more of the methods according to the invention and/or (c₂) second labeling means as defined in one or more of the methods according to the invention; and (d) first labeling means as defined in one or more of the methods according to the invention, and, optionally, one or more analyte antibodies, which are reactive with the one or more first and second antigenic molecules as defined in one or more of the methods according to the invention. In another embodiment the kit according to the present invention comprises (a) said first antigenic molecules labeled with a second labeling means, or (a) said first antigenic molecules immobilized to a solid support; and (b) said second antigenic molecules labeled with a first labeling means.

In still other embodiments the present invention provides the use of any of the methods of the kits according to the invention for the diagnosis of the presence or onset of a disease related to the insulin receptor family and/or for the identification of a pharmaceutically effective compound for the treatment and/or prophylaxis of a disease related to the insulin receptor family.

### Detailed Description of the Invention

Principally, the terms and phrases used in this application shall have the meaning of the general knowledge of the person skilled in the art. However, the following preferred meaning of specified terms and phrases may be noted:
The terms "polypeptide" and "protein" are used interchangeable throughout this application.

The term "sample" to be investigated according to the present invention shall preferably mean any sample which is essentially a liquid or suspension, preferably of biological and/or chemical origin. The sample can be obtained by well known techniques and may preferably mean isolated body fluids such as blood, plasma, serum, cerebrospinal fluid, saliva, urine, seminal liquid, tear fluids and others. The sample may further encompass nail clippings, faeces, other excrement, separated cells, cell homogenates, tissue homogenates, or organ homogenates obtained from an animal (e.g., mouse, rat, guinea pig, dog, pig, primates) or human individual. Tissue samples or organ samples may be obtained from any tissue or organ by, e.g., biopsy. Separated cells may be obtained from the body fluids or the tissues or organs by separating techniques such as centrifugation or cell sorting. Preferably, cell samples, tissue samples or organ samples are obtained from those cells, tissues or organs which express, contain, accumulate, concentrate or produce the antigenic molecules referred to herein. The sample may have been subject to treatment and/or modification, known to the person skilled in the art in order to allow storage or further processing in a method of the invention. For example, the person skilled in the art knows that the sample may be diluted in a suitable buffer, or, if the sample is derived from urine, any biotin contained in the sample need to be removed in order to avoid interference with accurate biotin determination, if biotin is used as a label means in the method of the invention. The sample may be selected from an individual suspected of the onset or presence of a dysfunctional condition selected from autoimmune disorders, growth, development or energy metabolism disorders, growth hormone binding protein disorder, growth hormone receptor disorder and growth hormone disorders. In one embodiment of the invention, the sample may comprise a known amount of analyte antibodies and/or one or more modulators, preferably, a known amount of both analyte antibodies and one or more modulator. This may be of particular relevance for the identification of modulators as hereinbefore described.

Generally, the term "antigenic molecule" according to the present invention means any molecule with which an analyte antibody can interact and which is capable of binding an (one or more) analyte antibody to form specific complexes comprising [analyte antibody-antigenic molecule]. The antigenic molecule may be natural or synthetic and modifications thereto are preferably such as to not detrimentally affect the binding properties in the methods according to the present invention.

The term "analyte antibodies" according to the present invention means any antibody capable of binding to any member of the IRF, respectively, capable of binding to one or more of the receptors selected from the group consisting of IR, IGF1R, IGF2R, IIHR, and IRRR as further outlined herein, whose presence is being quantitatively and/or qualitatively analyzed. The term "analyte antibody" may include a monoclonal antibody, a polyclonal antibody, a single chain antibody, a bispecific antibody or diabody, a bivalent antibody, a multispecific antibody, a synthetic antibody, an aptamer, a spiegelmer, a human or humanized antibody, and a fragment or variant thereof such as, e.g., Fab, Fv or scFv fragments, or a chemically modified derivative of any of these, e.g. antibody-drug conjugates, domain antibodies, nanobodies or antibody mimetica (DARPins, designed ankyrin repeat proteins). In specific embodiments of the present invention the term "analyte antibodies" means endogenous autoantibodies, therapeutic antibodies, and/or diagnostic antibodies.

The term "member of the insulin receptor family", respectively, "insulin receptor family" (IRF) according to the present invention means any polypeptide selected from the group consisting of the insulin receptor (IR), the insulin-like growth factor 1 receptor (IGF1R), the insulin-like growth factor 2 receptor (IGF2R), the insulin-IGF1 hybrid receptor (IIHR), the insulin receptor-related receptor (IRRR), any subunit, variant, analogue, derivative, and fragment thereof. Preferably, the IRF is of animal (e.g., mouse, rat, guinea pig, dog, pig, primates) or human origin, more preferred human.

In one embodiment of the methods of the invention the term "member of the insulin receptor family" or "insulin receptor family" (IRF) means the insulin receptor (IR) or any subunit, or any variant thereof. In another embodiment of the present invention only the said first antigenic molecules are selected from the insulin receptor (IR) or any subunit, or any variant thereof.

In a second embodiment of the methods of the invention the term "member of the insulin receptor family" or "insulin receptor family" (IRF) means the insulin-like growth factor 1 receptor (IGF1R) or any subunit, or any variant thereof. In another embodiment of the present invention only the said first antigenic molecules are selected from the insulin-like growth factor 1 receptor (IGF1R) or any subunit, or any variant thereof.

In a third embodiment of the methods of the invention the term "member of the insulin receptor family" or "insulin receptor family" (IRF) means the insulin-like growth factor 2 receptor (IGF2R) or any variant thereof. In another embodiment of the present invention only the said first antigenic molecules are selected from the insulin-like growth factor 2 receptor (IGF2R) or any variant thereof.

In a fourth embodiment of the methods of the invention the term "member of the insulin receptor family" or "insulin receptor family" (IRF) means the insulin-IGF1 hybrid receptor (IIHR) or any subunit, or any variant thereof. In another embodiment of the present invention only the said first antigenic molecules are selected from the insulin-IGF1 hybrid receptor (IIHR) or any subunit, or any variant thereof.

In a fifth embodiment of the methods of the invention the term "member of the insulin receptor family" or "insulin receptor family" (IRF) means the insulin receptor-related receptor (IRRR) or any subunit, or any variant thereof. In another embodiment of the present invention only the said first antigenic molecules are selected from the insulin receptor-related receptor (IRRR) or any subunit, or any variant thereof.

In a sixth embodiment of the methods of the invention the term "member of the insulin receptor family" or "insulin receptor family" (IRF) means any polypeptide selected from the insulin receptor (IR) or any subunit, or any variant thereof and the insulin-like growth factor 1 receptor (IGF1R) or any subunit, or any variant thereof.

In a seventh embodiment of the methods of the invention the term "member of the insulin receptor family" or "insulin receptor family" (IRF) means any polypeptide selected from the insulin receptor (IR) or any subunit, or any variant thereof and the insulin-like growth factor 2 receptor (IGF2R) or any variant thereof.

In an eighth embodiment of the methods of the invention the term "member of the insulin receptor family" or "insulin receptor family" (IRF) means any polypeptide selected from the insulin receptor (IR) or any subunit, or any variant thereof and the insulin-IGF1 hybrid receptor (IIHR) or any subunit, or any variant thereof.

In a ninth embodiment of the methods of the invention the term "member of the insulin receptor family" or "insulin receptor family" (IRF) means any polypeptide selected from the insulin receptor (IR) or any subunit, or any variant thereof and the insulin receptor-related receptor (IRRR) or any subunit, or any variant thereof.

In a tenth embodiment of the methods of the invention the term "member of the insulin receptor family" or "insulin receptor family" (IRF) means any polypeptide selected from the insulin-like growth factor 1 receptor (IGF1R) or any subunit, or any variant thereof and the insulin-like growth factor 2 receptor (IGF2R) or any variant thereof.

In an eleventh embodiment of the methods of the invention the term "member of the insulin receptor family" or "insulin receptor family" (IRF) means any polypeptide selected from the insulin-like growth factor 1 receptor (IGF1R) or any subunit, or any variant thereof and the insulin-IGF1 hybrid receptor (IIHR) or any subunit, or any variant thereof.

In a twelfth embodiment of the methods of the invention the term "member of the insulin receptor family" or "insulin receptor family" (IRF) means any polypeptide selected from the insulin-like growth factor 1 receptor (IGF1R) or any subunit, or any variant thereof and the insulin receptor-related receptor (IRRR) or any subunit, or any variant thereof.

In a thirteenth embodiment of the methods of the invention the term "member of the insulin receptor family" or "insulin receptor family" (IRF) means any polypeptide selected from the insulin-like growth factor 2 receptor (IGF2R) or any variant thereof and the insulin-IGF1 hybrid receptor (IIHR) or any subunit, or any variant thereof. In a fourteenth embodiment of the methods of the invention the term "member of the insulin receptor family" or "insulin receptor family" (IRF) means any polypeptide selected from the insulin-like growth factor 2 receptor (IGF2R) or any variant thereof and the insulin receptor-related receptor (IRRR) or any subunit, or any variant thereof.

In a fifteenth embodiment of the methods of the invention the term "member of the insulin receptor family" or "insulin receptor family" (IRF) means any polypeptide selected from the insulin-IGF1 hybrid receptor (IIHR) or any subunit, or any variant thereof and the insulin receptor-related receptor (IRRR) or any subunit, or any variant thereof.

Suitable polypeptides and their corresponding genes, which encode the members of the IRF, are well known to the skilled person in the art. Members of the IRF are commercially available from recombinant sources (e.g. from R&D Systems, Inc., Minneapolis, MN 55413, USA, OriGene Technologies, Inc., Rockville, MD 20850, USA) and are well known from protein and nucleic acid sequence databases, such as, e.g., EMBL, Genbank and others. Currently available database accession numbers for members of the IRF are given for the human species at the specific receptor proteins, however, the present invention shall not be understood to be limited thereto.

The term "insulin receptor" (IR) according to the present invention means any isolated polypeptide having a naturally occurring amino acid sequence or any variant thereof. The amino acid sequences and gene sequences encoding IR are well known to the skilled person, e.g., from entries in sequence databases such as UniProtKB, e.g., P06213 (1382 amino acid length from *Homo sapiens*). In another embodiment of the present invention the IR is modified for use in in vitro analysis so that it does not comprise a functional intact tyrosine kinase domain or may completely lack the intracellular tyrosine kinase domain due to deletion, e.g., by use of recombinant technologies in protein modification, which are well known to the person skilled in the art. In still another embodiment of the present invention, IR means an IR variant, which exhibits pathogenic or dysfunctional prevalence in an animal (e.g., mouse, rat, guinea pig, dog, pig, primates) or human subject. In yet another embodiment of the present invention the IR is embedded in a membrane environment.

The term "insulin-like growth factor 1 receptor" (IGF1R) according to the present invention means any isolated polypeptide having a naturally occurring amino acid sequence or any variant thereof. The amino acid sequence and gene sequence encoding IGF1R are well known to the skilled person, e.g., from entries in sequence databases such as UniProtKB, e.g., P08069 (1367 amino acid length from *Homo sapiens*) or C9J5X1 (1366 amino acid length from *Homo sapiens*). In another embodiment of the present invention the IGF1R is modified for use in in vitro analysis so that it does not comprise a functional intact tyrosine kinase domain or may completely lack the intracellular tyrosine kinase domain due to deletion, e.g., by use of recombinant technologies in protein modification, which are well known to the person skilled in the art. In still another embodiment of the present invention, IGF1R means an IGF1R variant, which exhibits pathogenic or dysfunctional prevalence in an animal (e.g., mouse, rat, guinea pig, dog, pig, primates) or human subject. In yet another embodiment of the present invention the IGF1R is embedded in a membrane environment.

The term "insulin-like growth factor 2 receptor" (IGF2R) according to the present invention means any isolated polypeptide having a naturally occurring amino acid sequence or any variant thereof. The amino acid sequence and gene sequence encoding IGF2R are well known to the skilled person, e.g., from entries in sequence databases such as UniProtKB, e.g., P11717 (2491 amino acid length from *Homo sapiens*). In another embodiment of the present invention, IGF2R means an IGF2R variant, which exhibits pathogenic or dysfunctional prevalence in an animal (e.g., mouse, rat, guinea pig, dog, pig, primates) or human subject. In still another embodiment of the present invention the IGF2R is embedded in a membrane environment.

The term "insulin-IGF-1 hybrid receptor" (IIHR) according to the present invention means any isolated polypeptide having a naturally occurring amino acid sequence or any variant thereof. The amino acid sequence and gene sequence encoding IIHR are well known to the skilled person. In another embodiment of the present invention the IIHR is modified for use in in vitro analysis so that it does not comprise a functional intact tyrosine kinase domain or may completely lack the intracellular tyrosine kinase domain due to deletion, e.g., by use of recombinant technologies in protein modification, which are well known to the person skilled in the art. In still another embodiment of the present invention, IIHR means an IIHR variant, which exhibits pathogenic or dysfunctional prevalence in an animal (e.g., mouse, rat, guinea pig, dog, pig, primates) or human subject. In yet another embodiment of the present invention the IIHR is embedded in a membrane environment.

The term "insulin-receptor related receptor" (IRRR) according to the present invention means any isolated polypeptide having a naturally occurring amino acid sequence or any variant thereof. The amino acid sequence and gene sequence encoding IRRR are well known to the skilled person, e.g., from entries in sequence databases such as UniProtKB, e.g., P14616 (1297 amino acid length from *Homo sapiens*). In another embodiment of the present invention the IRRR is modified for use in in vitro analysis so that it does not comprise a functional intact tyrosine kinase domain or may completely lack the intracellular tyrosine kinase domain due to deletion, e.g., by use of recombinant technologies in protein modification, which are well known to the person skilled in the art. In still another embodiment of the present invention, IRRR means an IRRR variant, which exhibits pathogenic or dysfunctional prevalence in an animal (e.g., mouse, rat, guinea pig, dog, pig, primates) or human subject. In yet another embodiment of the present invention the IRRR is embedded in a membrane environment.

The term "variant" according to the present invention means any fragment, analog, derivative, fusion protein, subunit or subunit chain of a molecule mentioned. Preferably, the variant may have at least essentially the same biological properties as the respective polypeptides or proteins mentioned, except for the tyrosine kinase activity, immobilizing features and/or labeling features. Moreover, it is to be understood that the term "variant" according to the present invention shall include an amino acid sequence which differs due to at least one amino acid substitution, modification, deletion and/or addition, wherein the amino acid sequence of the variant is still, preferably, at least about 50%, at least about 60%, at least about 70%, at least about 80%, at least about 85%, at least about 90%, at least about 92%, at least about 95%, at least about 97%, at least about 98%, or at least about 99% identical with the amino sequence of the respective polypeptide or protein mentioned, preferably over the entire length of the specific polypeptide or protein. Variants may be allelic variants or any other species specific homologs, paralogs, or orthologs. Moreover, the variants referred to herein include fragments of the respective polypeptides or proteins mentioned hereinbefore from the IRF, provided these fragments have essentially the same biological properties. Furthermore, the variants referred to herein include fusion proteins of the respective polypeptides or proteins mentioned hereinbefore from the IRF with polypeptides, which are suitable as immobilization means, labeling means, or label, provided such fusion protein essentially maintains the same biological properties of the respective polypeptide or protein mentioned hereinbefore from the IRF. Variants may further include modifications of the said polypeptides or proteins by glycosylation or any other chemical or enzymatic modification, provided these variants have essentially the same biological properties as referred to above.

The variants according to the invention may include so-called 'silent' substitutions, additions are deletions which do not alter or substantially alter the biological activity. More particularly, the variants according to the present invention may have been modified with respect to one or more of the amino acid residues, which are substituted by a conserved or non-conserved amino acid residue, preferably a conserved amino acid residue, or such ones in which one or more of the amino acid resides may include a substituted radical. Such variants are deemed to be within the scope of the teachings herein. Most typically, variants are those that vary by conservative amino acid substitutions. Such substitutions are those that substitute a given amino acid in a polypeptide by another amino acid of similar chemical characteristics. In this regard are understood as conservative substitutions the replacements, one for another, among the small aliphatic amino acids A, V, L and I; among the hydroxyl residues S and T; among the acidic residues D and E; among the amide residues N and Q; among the basic residues K and R; and among the aromatic residues F and Y (according to the single letter code of amino acids of the IUPAC nomenclature).

In one embodiment of the present invention, a "variant" shares essentially the same biological properties. In another embodiment of the present invention a "variant" of a member of the IRF is used, which exhibits pathogenic or dysfunctional prevalence, i.e., which exhibits a disease related to the insulin receptor family in an animal (e.g., mouse, rat, guinea pig, dog, pig, primates) or human subject.

According to another embodiment of the present invention the one or more members of the IRF are modified so that they do not comprise a functional intact tyrosine kinase domain or completely lack the intracellular tyrosine kinase domain, e.g., by means of recombinant technologies in protein modification, which are well known to the person skilled in the art.

The term "biological properties" in accordance with the understanding of the present invention means the binding properties of the respective molecule mentioned. In case of a specific member of the IRF it may be its ability to form a complex with insulin, IGF-1, and/or IGF-2 under suitable conditions. For the purpose of the present invention, it is understood that the signaling property of any member of the IRF with respect to the tyrosine kinase activity is expressly excluded. Optionally, the term "biological properties" may further include certain specific immunological properties of polypeptides, e.g., if they are specifically detectable by the same ELISA method. Particularly, a member of the IRF exhibits essentially the same "biological properties" like a variant thereof, if both (a) can interact with the analyte antibodies, (b) are detectable by the same ELISA method and/or (c) are detectable by the detection method according to the present invention.

In another preferred embodiment of the invention the one or more first antigenic molecules and the one or more second antigenic molecules are one or more polypeptides selected from the group consisting of IR, IGF1R, IGF2R, IIHR, and IRRR.

The term "providing prior to step (c) of the method of detecting autoantibodies", respectively, "providing prior to step (d) of the method for the identification of modulators" according to the present invention shall mean prior to contacting the said antigenic molecules and the said analyte antibodies in a method of the present invention.

The term "detecting the presence and/or the binding properties" according to the present invention means determining the amount or concentration, preferably quantitatively. The measuring can be done directly or indirectly. Direct measuring relates to measuring the amount or concentration of one or more of the reaction educts and/or the reaction products based on a signal which is obtained from the one or more reaction educts and/or the reaction products itself/themselves and the intensity of which directly correlates with the number of molecules of the one or more reaction educts and/or the reaction products in the reaction volume. Such a signal may be obtained, e.g., by measuring the intensity or value of a specific physical or chemical property of the one or more reaction educts and/or reaction products. Indirect measuring includes measuring of a signal obtained from a secondary component (i.e. a component not being the reaction educt or reaction product itself) or a biological read out system, referred to as "label means" in this specification, e.g., of measurable cellular or transmembrane responses, ligands, or enzymatic reaction products, e.g. by means of fluorophors, chromophors, ion concentrations, which suitably is performed by means of optical, electrical and/or electronical equipment. For measurement of enzymatic reaction products, preferably the amount of substrate is saturating. Optionally, the substrate may also be labeled with a detectable label prior to the reaction. Preferably, the reaction partners are contacted with the substrate for an adequate period of time, which corresponds to the time necessary for a detectable amount of the one or more reaction products to be produced such as a measurable signal. Instead of measuring the amount or concentration of the one or more reaction products, the time necessary for appearance of a given (e.g. detectable) amount or concentration of the one or more reaction products can be measured.

According to the present invention, "detecting the presence and/or the binding properties" can be achieved by all means for determining the amount of a reaction educt and/or reaction product known to the skilled person. Said means comprise immunoassay devices and methods which may utilize labeled molecules in various sandwich, competition, or other assay formats. Said assays will develop a signal which is indicative for the presence or absence of the reaction educts and/or the reaction products. Moreover, the signal strength can, preferably, be correlated directly or indirectly (e.g. reverse-proportional) to the amount of reaction educts and/or the reaction products in the reaction volume. Said methods comprise, preferably, biosensors, optical devices coupled to immunoassays, biochips, analytical devices such as spectrometers or chromatography devices. Further, methods include ELISA-based methods using, optionally pre-treated or pre-coated, micro-plates, micro-arrays, or tube-arrays, fully-automated or robotic immunoassays (available, e.g., on Roche-Elecsys^{™}, Abbott-AxSYM^{™} or Brahms Kryptor^{™} analyzer systems). Preferably, "detecting the presence and/or the binding properties" comprises the steps which will allow bringing the reaction partners together for an adequate period of time.

The term "binding" according to the present invention includes both covalent and non-covalent binding. The term "specific binding" means a binding affinity of at least 3 times higher, preferably of at least about 10 times higher and more preferably of at least about 50 times higher than the binding affinity to other molecules. In another embodiment of the present invention the term "binding" shall mean the binding of binding partners in an in vitro binding assay under suitable conditions, preferably under conditions according to the assay manufacturer's instructions or according to the methods essentially as defined hereinafter in the Example section. Generally, "binding" means a binding affinity (K_{D} - which means the quotient of dissociation constant to association constant) of about 10⁻¹⁴ M to 10⁻⁷ M, preferably of about 10⁻¹³ M to 10⁻⁹ M.

A "modulator" according to the present invention includes any biological or chemical compound, a macromolecule (e.g. larger than about 5 kDa), a small molecule (e.g., smaller than about 5 kDa or even smaller than about 800 Da), an isolated compound or a mixture of compounds, an extract or homogenates of tissue or organ origin, or a compound of synthetic origin comprising, e.g., one or more peptides, polypeptides (e.g., polyclonal or monoclonal antibodies, including single chain antibodies, diabodies, multispecific antibodies, humanized antibodies, hybrid antibodies, or fragments thereof, such as Fv, Fab and F(ab)₂ fragments), aptamers, spiegelmers, nucleic acids, and/or small molecules.

The term "antigenic molecule embedded in a membrane environment" according to the present invention means that the said antigenic molecule - which is in case of the IRF a transmembrane protein - is provided in a model membrane or the like, which includes any suitable synthetic, natural or artificial environment of a, e.g., cellular, membrane, vesicular, micellar or liposomal structure, whereby the said antigenic molecule is able to interact with the aforementioned analyte antibodies and thus complex formation.

Assay methods for proteins embedded in a membrane environment are well known to the skilled person, e.g., from technologies and assays which use artificial or biomimetical membranes or lipid bilayers (herein "model membranes"). Model membranes are widely used for investigating membrane proteins and many of them are suitable for the performance of the methods of the present invention, particularly, if denaturation of the said antigenic molecule can be avoided.

The term "model membrane" according to the present invention encompasses, e.g., any liposome or vesicle, such as artificially prepared vesicles comprising one, two or more lipid layers in spherical geometry. Such liposomes and vesicles are used as vehicles for the transport of lipids, proteins and small molecules; therefore they are used in the administration of pharmaceuticals. Vesicles or liposomes can easily be prepared by disrupting biological membranes from cells cultures, tissues, organs, or subcellular structures (e.g. nucleus, Golgi, endoplasmatic reticulum, and mitochondria), e.g., by sonication and/or extrusion) and subsequent self-reassembling of the lipid structures, whereby labeling with dyes or labeled polypeptides suitable in RET is easily achievable. Further "model membranes" may comprise lipid bilayers which have been synthetically assembled in vitro. They can be made from one or more synthetic and/or natural lipids.

The term "model membranes" according to the present invention may further include, e.g., black lipid membranes (BLM), vesicles, lipid bilayers, liposomes, micelles, bicelles, hybrid bilayers (e.g. comprising a hydrophobic monolayer and a lipid monolayer), and nanodiscs, which may be anchored, supported or tethered to a solid phase or solid substrate, and which may, optionally, be provided with a spacer or cushion (e.g., polyethylene glycols, oligonucleotides, peptides, polypeptides (e.g., streptavidin), hydrogels and others) which may allow to maintain a distance of the membrane and/or the aforementioned antigenic molecule to the solid substrate. Preferably, the spacer or cushion is a hydrophilic molecule. In contrast to a vesicle or a cell membrane, the aforementioned supported bilayer may have a planar structure sitting on a solid support. Therefore, only the upper face of the bilayer is exposed to the solution. For example, the preparation of proteoliposomes is well known from European patent application EP 1992688 A1, of which the liposome preparation methods as disclosed in any of examples 1 to 20 are herewith incorporated by reference.

The term "micelles" according to the present invention means another type of model membranes which lack a lipid bilayer. In aqueous solutions, micelles are assemblies of amphipathic molecules (e.g., detergents) with their hydrophilic heads exposed to solvent and their hydrophobic tails in the center. Micelles are able to solubilize membrane proteins by partially encapsulating them and shielding their hydrophobic surfaces from solvent. The term "bicelles" means still another type of model membranes which are typically made of two lipids, one of which forms a lipid bilayer while the other forms an amphipathic, micelle-like assembly shielding the bilayer center from surround solvent molecules. The term "nanodiscs" means a segment of a bilayer encapsulated by an amphipathic protein coat, a lipid or detergent layer. Membrane proteins can be incorporated into and solubilized by nanodiscs.

The term "disease related to the insulin receptor family" according to the present invention preferably means any dysfunction which is related to one or more polypeptides selected from the group consisting of IR, IGF1R, IGF2R, IIHR, and IRRR, more preferably of one, two, or three selected polypeptides of the said IRF. The term "disease related to the insulin receptor family" according to the present invention encompasses autoimmune disorders (such as, e.g., insulin dependent diabetes mellitus (IDDM), Morbus Basedow, Graves Orbitopathy) and also disorders and dysfunctions such as obesity, neurological disorders, growth disorders, cancer generation and development (including breast, colon, ovarian, prostate, lung), and other cellular proliferative disorders and dysfunctions, whether of autoimmune origin or not, preferably of autoimmune origin.

The term "immobilizing means" according to the present invention means any reagent and/or process, which is suitable to immobilize the said first antigenic molecules to a solid support according to the knowledge of the person skilled in the art. With respect to the kind of a solid support and conditions employed in accordance with the present invention, the solid support and conditions do generally not fundamentally differ from conventionally used solid supports and conditions employed in known immunoassay techniques. A solid support for use according to the present invention can comprise an ELISA plate as currently employed in known ELISA techniques, or may employ any other suitable support for use in the present invention, such as micro-titer plates (having 96, 384, 1536, or 3456 wells or more) or parts thereof, tubes, particles, magnetic beads, nitrocellulose or the like. Materials suitable as solid support which can be used in accordance with the teachings of the present invention are well known in the art and include, e.g., commercially available column materials, polystyrene beads and other carriers, latex beads, magnetic beads, colloidal metal, glass surfaces and chips for use in protein microchip technologies, silanylated surfaces and chips for use in protein microchip technologies, silicon surfaces and chips for use in protein microchip technologies, nitrocellulose carriers, cellulose carriers, membranes, model membranes, stabilized liposomes or cells (e.g., duracytes), wells and walls of reaction trays or microtiter plates, plastic tubes etc.

The antigenic molecules as hereinbefore defined may be immobilized to any carrier which is known to the person skilled in the art. Examples of such carriers include glass, polystyrene, polyvinyl chloride, polypropylene, polyethylene, polycarbonate, dextran, nylon, amylose, natural and modified cellulose, polyacrylamide, agarose, magnetite, and gold. The carrier can be either soluble or insoluble, in case of an insoluble carrier; the carrier is a solid or colloid and may, optionally, be provided as suspension.

Suitable methods for immobilizing said antigenic molecules are well known and include, but are not limited to ionic, hydrophobic, covalent interactions and the like. It may also be suitable to use solid immobilization means in suspension according to the present invention, wherein the carrier is provided in suspension, e.g. a microbead or microsphere consisting of different microbeads or microspheres, optionally labeled, both carrying each different molecules. Methods of producing such suspensions, e.g., based on solid-phase chemistry and photo-labile protective groups, are, e.g., known from US 5,744,305.

The term "labeling" according to the present invention means labeling by direct or indirect methods. Direct labeling involves coupling of the label directly (covalently or non-covalently) to the molecule to be labeled. Indirect labeling involves binding (covalently or non-covalently) of a second ligand to the molecule to be labeled. Such second ligand should specifically bind to the molecule to be labeled with an at least 3-fold higher, preferably at least 10-fold, and more preferred at least 50-fold higher affinity under assay conditions. Said second ligand may be coupled with a suitable label means and/or may bind a third ligand binding to the second ligand. The use of second, third, or even higher order ligands is often used to increase the signal. Suitable second and higher order ligands may include antibodies, secondary antibodies, and the well-known streptavidin-biotin system (Vector Laboratories, Inc.). Furthermore, the molecule to be labeled or the substrate may also be "tagged" with one or more tags known in the art. Such tags may then be targets for higher order ligands. Suitable tags include biotin, digoxygenin, His-Tag, Glutathion-S-Transferase, FLAG-tag (N-DYKDDDDK-C), green fluorescence protein (GFP), myc-tag, influenza a virus haemagglutinin (HA), maltose binding protein, and others. In the case of a peptide or polypeptide, the tag is generally located at or close to the N-terminus and/or C-terminus.

Furthermore, the molecule to be labeled or the substrate may also be provided with a suitable "spacer" known in the art in order to avoid in case of bulky molecules any limitations with respect to the binding properties due to spatial constrictions.

The term "first labeling means" according to the invention shall preferably mean any direct or indirect detectable labeling means selected from the group of enzymatic labels, isotopic or radioactive labels, chemoluminescent labels, bioluminescent labels, fluorescent labels, magnetic labels (e.g. "magnetic beads", including paramagnetic and superparamagnetic labels), dye labels (chromophors), and others known in the art. Suitable labels are detectable by an appropriate detection method known in the art. Suitable labels may further include gold particles, latex beads, acridan ester, luminol, and ruthenium. Preferably suitable are non-radioactive labels.

Enzymatically active labels include, e.g., horseradish peroxidase, alkaline phosphatase, beta-galactosidase, luciferase, and derivatives thereof. Suitable substrates for detection include di-amino-benzidine (DAB), 3, 3'-5, 5'-tetramethylbenzidine, NBT-BCIP (4-nitro blue tetrazolium chloride and 5-bromo-4-chloro-3-indolyl-phosphate, CDP-Star^{™} (Amersham Biosciences), ECL^{™} (Amersham Biosciences) and others known in the art.

A suitable enzyme-substrate combination may result in increase or decrease of a colored reaction product (chromophor), fluorescence, or chemo- or bioluminescence, which can be measured according to methods known in the art (e.g. using a photometer, a photo-multiplier, and a light-sensitive film or camera system). The same principles apply for measuring the endpoint or performance or development of an enzymatic reaction.

Suitable fluorescence labels include fluorescent dyes and proteins (such as GFP and its derivatives), Cy3, Cy5, Texas Red, fluorescein, and the Alexa dyes (e.g. Alexa 568). Further suitable fluorescent labels are commercially available e.g. from Molecular Probes (Oregon, USA). Also the use of quantum dots as fluorescent labels is encompassed. Examples of fluorescent proteins include, but are not limited to, green, yellow, cyan, blue, and red fluorescent proteins.

Suitable chemoluminescence or bioluminescence labels include, but are not limited to prokaryotic (e.g., bacterial lux-encoded) or eukaryotic (e.g., firefly luc-encoded) luciferases, as well as variants possessing varied or altered optical properties, such as luciferases that produce different colors of light, e.g. derived from *Photinus pyralis,* from the sponge *Suberities domuncula,* and the *Mycena* fungi. Furthermore, photoproteins, e.g., calcium-activated photoproteins and their specifically designed variants may be suitable, which are capable of producing light typically in the range of 200 nm to 1100 nm, or in the visible spectrum (i.e., between approximately 350 nm and 800 nm), e.g., obelin from the marine polyp *Obelia longissima,* or Aequorin, e.g., from the luminescent jellyfish *Aequorea victoria* or from other organisms may be suitable, optionally in a membrane.

Suitable radioactive labels include ³⁵S, ¹²⁵I, ³²P, ³³P and the like. A radioactive label can be detected by any method known and appropriate, e.g. a light-sensitive film or a phosphor imager.

Suitable detection methods according to the present invention also include precipitation (particularly immunoprecipitation), electrochemiluminescence (electrically generated chemiluminescence), biolominescence, RIA (radioimmunoassay), ELISA (enzyme-linked immunosorbent assay), sandwich enzyme immune tests, sandwich immunoassays (ECLIA), dissociation-enhanced lanthanide fluorescent Immunoassay (DELFIA^{™}, PerkinElmer Inc., USA), scintillation proximity assay (SPA), turbidimetry, nephelometry, latex-enhanced turbidimetry or nephelometry, latex agglutination assay, or solid phase immune assays.

Further methods known in the art (such as gel electrophoresis, 2D gel electrophoresis, SDS polyacrylamid gel electrophoresis (SDS-PAGE), Western Blotting, and mass spectrometry), can optionally be used in combination with the labeling or other detection methods as described above.

The one or more antigenic molecules according to the invention may be directly or indirectly provided with the labeling means, substantially as hereinbefore described. The term "second labeling means" according to the invention shall preferably mean any direct or indirect detectable labeling means selected from the group of chemoluminescent labels, bioluminescent labels, fluorescent labels, dye labels (chromophors), and others known in the art. Suitable second labeling means are detectable by an appropriate detection method known in the art based on resonance energy transfer (RET) principle. Preferably, "second labeling means" include labeling means suitable for fluorescence resonance energy transfer (FRET), bioluminescence resonance energy transfer (BRET), or chemoluminescence resonance energy transfer (CRET) as known to the person skilled in the art. The selection of a suitable second labeling means will consider the kind of the first labeling means in order to safeguard that the RET signal is detectable and are known to the person skilled in the art. Furthermore, the RET signal generation and selection of suitable labeling means provides the person skilled in the art with additional information about the kind and kinetics of complex formation and the structural features of the complexes formed.

Sequences:
Seq. ID No. 1 Primer P1 (33mer)
Seq. ID No. 2 Primer P2 (34mer)
Seq. ID No. 3 Primer P3 (35mer)
Seq. ID No. 4 Primer P4 (33mer)
Seq. ID No. 5 DNA encoding amino acids 2-551 from firefly luciferase
Seq. ID No. 6 amino acid sequence 2-551 from firefly luciferase
Seq. ID No. 7 DNA encoding amino acids 1-1367 from human IGF1R
Seq. ID No. 8 amino acid sequence 1-1367 from human IGF1R
Seq. ID No. 9 DNA encoding amino acids 1-1919 from human IGF1R-luc fusion Seq. ID No. 10 amino acid sequence 1-1919 from human IGF1R-luc fusion
Seq. ID No. 11 DNA encoding amino acids 1-1370 from human insulin receptor Seq. ID No. 12 amino acid sequence 1-1370 from human insulin receptor (IR)
Seq. ID No. 13 DNA encoding amino acids 1-1922 from human IR-luc fusion
Seq. ID No. 14 amino acid sequence 1-1922 from human insulin receptor-luc fusion
Seq. ID No. 15 DNA encoding amino acids 1-927 from the extracellular domain of human IGF1R (ECDhIGFIR)
Seq. ID No. 16 amino acid sequence 1-927 from the extracellular domain of human IGF1R (ECDhIGF1R)
Seq. ID No. 17 Primer P5 (33mer)
Seq. ID No. 18 Primer P6 (33mer)
Seq. ID No. 19 Primer P7 (36mer)
Seq. ID No. 20 Primer P8 (83mer)

The present invention will now be illustrated by the following examples, which shall be understood not to limit the scope of the present invention in any way.

Experimental Procedures:
Materials:
   DNA primers (P1-P8) were obtained from BioTeZ Berlin Buch GmbH (Berlin, Germany); pSP-luc+NF vector was obtained from Promega GmbH (Mannheim, Germany); pIRESneo vector was obtained from Clontech (Palo Alto, CA, USA); vector pCR-XL-TOPO-IR was obtained from ImaGenes GmbH (Berlin, Germany); vector pFastBac1 was obtained from Invitrogen; Goat anti human IgG (SIGMA) was labeled with Acridinium NHS Ester (Cayman Chemical Company, Ann Arbor, MI, USA); IGF1 was obtained from Acris Antibodies (Herford, Germany); Insulin was obtained from Sigma-Aldrich; anti-IGF1R (Tyr1165/Tyr1166) antibody was obtained from Novus Biologicals (Edinburgh, UK); CellTiter-Glo luminescent cell viability assay kit was obtained from Promega GmbH; High Five insect cells were purchased from Invitrogen; Polystyrene tubes coated with PGA14 antibody (Selenotest LIA) were obtained from ICI immunochemical intelligence GmbH (Berlin, Germany). If not otherwise stated, all other reagents and chemicals were obtained from Sigma-Aldrich Chemie GmbH (Munich, Germany) or Merck KGaA (Darmstadt, Germany); enzymes were obtained from Promega or New England Biolabs (Ipswich, MA, USA).

### Example 1: Construction of fusion proteins

### Example 1A: Construction of an IGF1R-luciferase fusion protein

The DNA (Seq. ID No. 5) encoding amino acids 2-551 from firefly luciferase (Seq. ID No. 6 on pSP-luc+NF) was amplified by PCR using primers P1 (Seq. ID No. 1) and P2 (Seq. ID No. 2) containing EcoRI and BamHI restriction sites, respectively. pIRESneo was digested with EcoRI and BamHI restriction endonucleases; the obtained fragment was replaced with the DNA encoding firefly luciferase obtained from the aforementioned PCR resulting in plasmid pIRESneo-Luc.

The DNA (Seq. ID No. 7) encoding amino acids 1-1367 from human IGF1R (Seq. ID No. 8) was amplified by PCR using primers P3 (Seq. ID No. 3) and P4 (Seq. ID No. 4) containing NotI and EcoRI restriction sites, respectively. pIRESneo-Luc was digested with NotI and BamHI restriction endonucleases and the obtained fragment was replaced with the DNA sequence encoding human IGF1R obtained from the aforementioned PCR resulting in vector pIRESneo-IGF1R-Luc containing Seq. ID No. 9 encoding Seq. ID No. 10.

### Example 1B: Construction of an IR-luciferase fusion protein

The DNA (Seq. ID No. 11 of pCR-XL-TOPO-IR) encoding amino acids 1-1370 from human IR (Seq. ID No. 12) was amplified by PCR using primers P5 (Seq. ID No. 17) and P6 (Seq. ID No. 18) containing HpaI and MfeI restriction sites, respectively. pIRESneo-Luc was digested with EcoRV and EcoRI restriction endonucleases and the obtained fragment was replaced with the DNA sequence encoding human IR obtained from the aforementioned PCR resulting in vector pIRESneo-IR-Luc containing Seq. ID No. 13 encoding Seq. ID No. 14.

Example 1C: Construction of a fusion protein of the extracellular domain of IGF1R (ECDhIGF1R) and the 16 amino acid epitope recognized by PGA14 antibodies The DNA (Seq. ID No. 15) encoding 927 amino acids of the extracellular domain of the human IGF1 receptor (Seq. ID No. 16, ECDhIGF1R) was amplified by PCR using primers P7 (Seq. ID No. 19) and P8 (Seq. ID No. 20) containing BamHI and HindIII restriction sites, respectively (P8 containing the coding sequence for the 16 amino acid epitope recognized by PGA14 antibodies). pFastBac1 vector was digested with BamHI and HindIII restriction endonucleases and the obtained fragment was replaced with the DNA sequence encoding the ECD of hIGFIR obtained from the aforementioned PCR resulting in vector pFastBac1-IGF1R-PGA14tag.

### Example 2: Generation of IGF1R-Luc and IR-Luc fusion protein producing cells

### Example 2A: Generation of IGF1R-Luc and IR-Luc producing HEK 293

HEK 293 cells were grown in DMEM supplemented with 10% fetal bovine serum. Cells were cultivated in a 5% CO2 atmosphere at 37° C. HEK 293 cells were transfected with pIRESneo-IGF1R-Luc vector or pIRESneo-IR-Luc vector using FuGENE6 transfection reagent (obtained from Roche Deutschland Holding GmbH, Grenzach-Wyhlen, Germany) according to the manufacturer's instruction. 48 hours after transfection, selection was started with 0.8 mg/ml G418 (Gibco^{™} BRL, Invitrogen). Stable clones expressing high levels of fusion protein were selected.

### Example 2B: Generation of recombinant baculovirus expressing ECDhIGF1R-PGA14tag fusion protein

The ECDhIGF1R-PGA14tag sequence obtained from example 1C was transferred to bacmid DNA by site-specific recombination in bacteria. The bacmid was then used to generate a fully recombinant baculovirus in Sf9 insect cells according to the protocols supplied by the manufacturer (Bac-to-Bac expression system manual, Invitrogen).

### Example 2C: Production of IGF1R-PGA14tag fusion protein

Suspension High Five insect cells were grown in Express Five serum-free medium to density 2x10e6 cells/ml. Cells were then infected with recombinant IGF1R-PGA14tag-baculovirus at a multiplicity of infection (MOI) of 1. 72 hours post-infection cell medium was collected and stored at -80C°.

### Example 3: Control of expression products

### Example 3A: Interaction of IGF1R-Luc cells with IGF1

Confluent HEK 293 cells grown in a 96 well plate were washed with DMEM/F12 medium and incubated with acridinium labeled IGF1 (about 1.0e06 RLU/well) diluted in 100 µl of DMEM/F12, 0.1% BSA for 3 h at 37° C in a 5% CO₂ atmosphere. In further experiments unlabelled IGF1 (0.3 mg/ml) was added together with acridinium labeled IGF1 to determine unspecific binding. After incubation cells were washed with DMEM, resuspended in PBS containing 2% triton X-100 and obtained lysates were measured in a luminometer Berthold Technologie AutoLumat Plus LB 953 for 10 sec. Each value represents the mean value (RLU) +/- standard deviation (SD) of duplicate measurements (relative light units (RLU) x 1000).

| **Table 1** | **Wildtype HEK 293** | **IGF1R-Luc HEK 293** |
|---|---|---|
| Labeled IGF1 | 27 +/- 4 | 55+/-7 |
| Labeled IGF1 + excess IGF1 | 11 +/- 1 | 12+/-1 |

This result indicates that IGF1R-luc fusion proteins are correctly processed and expressed at the cell membrane.

### Example 3B: Interaction of IR-Luc cells with insulin

Confluent HEK 293 cells grown in a 96 well plate were washed with DMEM/F12 medium and incubated with acridinium labeled insulin about 1.0e06 RLU/well) diluted in 100 µl of DMEM/F12, 0.1% BSA for 3 h at 37° C in a 5% CO₂ atmosphere. In further experiments unlabelled insulin (0.3 mg/ml) was added together with acridinium labeled insulin to determine unspecific binding. After incubation the cells were washed with DMEM, resuspended in PBS containing 2% triton X-100 and obtained lysates were measured in a luminometer Berthold Technologie AutoLumat Plus LB 953 for 10 sec. Each value represents the mean value (RLU) +/- standard deviation (SD) of duplicate measurements (RLU x 1000).

| **Table 2** | **Wild type HEK 293** | **IR-Luc HEK 293** |
|---|---|---|
| Labeled insulin | 32 +/- 2 | 78 +/- 6 |
| Labeled insulin + excess insulin | 13 +/- 2 | 17 +/- 3 |

This result indicates that IR-luc fusion proteins are correctly processed and expressed at the cell membrane.

### Example 4: Preparation of IGF1R-Luc and IR-Luc cell extract

Confluent HEK 293 cells (producing either IGF1R-Luc or IR-Luc) grown in a 75 cm² plate were resuspended by scraping into PBS and were washed in the same buffer by centrifugation at 2500 rpm. The resulting cells were lysed in 0.5 ml buffer containing 20 mM HEPES-NaOH pH 7.5, 50 mM NaCl, 1% Triton X-100, 10% glycerol. The suspension was centrifuged at 5,000 rpm for 15 min and the supernatant was collected and stored at -80 C.

### Example 5: Immunoprecipitation assay for IGF1R autoantibodies

The IGF1R-Luc cell extract was diluted 10 times with buffer containing 20 mM HEPES-NaOH pH 7.5, 50 mM NaCl, 1% Triton X-100, 10% glycerol, 5 mg/ml BSA. For immunoprecipitation, 100 µl of diluted extract (about 1.0e07 RLU) was mixed with 10 µl of a sample (serum probe) and incubated overnight at 4°C. Immune complexes were precipitated by addition of 100 µl of 10% protein A-sepharose suspension in same buffer for 1 h at room temperature with shaking. Protein A-sepharose was pelleted and washed 3 times with 1 ml of washing buffer (10 mM Tris-HCl, pH 7.5, 60 mM NaCl, 0.02 % Tween 20). Finally, luciferase activity was measured in a Berthold luminometer (AutoLumat Plus LB 953) for 10 sec. Results were expressed as RLU bound. Each value represents the mean value of duplicate measurements. The ability of crude sera and isolated IgG preparations from the same sera IgG to immunoprecipitate the IGF1 receptor was compared.

| **Table 3** | **Serum [RLU]** | **RLU-serum [% of max]** | **IgG [RLU]** | **RLU-IgG [% of max]** |
|---|---|---|---|---|
| 1 | 417 | 0 | 238 | 0 |
| 2 | 619 | 0 | 181 | 0 |
| 3 | 781 | 1 | 392 | 1 |
| 4 | 669 | 0 | 414 | 1 |
| 5 | 348 | 0 | 293 | 0 |
| 6 | 121585 | 83 | 70472 | 92 |
| 7 | 145972 | 100 | 76178 | 100 |
| 8 | 130683 | 90 | 62441 | 82 |
| 9 | 55840 | 38 | 23002 | 30 |
| 10 | 131560 | 90 | 49699 | 65 |

### Table 4: Dilution, recovery and stability of the IGF1R autoantibody assay.

(A) Five different sera containing autoantibodies were diluted with incubation buffer and measured in assay as described in example 5. (B) Five sera with IGF1R autoantibodies, five sera without IGF1R autoantibodies and their mixtures (1:1) were analyzed in assay as described in example 5. (C) Mix of five sera was incubated at room temperature (RT) and 4°C for given periods of time and analyzed in IGF1R autoantibodies assay as described in example 5.

Table 4A: Dilution experiments for the IGF1R autoantibodies assay.

Five different sera containing autoantibodies were diluted with incubation buffer and measured in assay as described in example 5. Bound luciferase activity was measured as previously described. Each value represents the mean value of duplicate measurements (RLU x 1000).

| **Table 4A Sera (%)** | **Serum 1** | **Serum 2** | **Serum 3** | **Serum 4** | **Serum 5** |
|---|---|---|---|---|---|
| 100 | 76,3 | 93,7 | 71,8 | 41,3 | 86,6 |
| 50 | 50,2 | 54,6 | 39,6 | 21,5 | 51,8 |
| 25 | 27,1 | 30,1 | 24,2 | 13,9 | 27,6 |
| 10 | 11,6 | 13,9 | 11,4 | 6,6 | 12,2 |

Table 4B: Recovery experiments for the IGF1R autoantibodies assay. Five sera without IGF1R autoantibodies (A), five sera with IGF1R autoantibodies (B) and their mixtures (1:1) were analyzed in the assay as described in example 5. Bound luciferase activity was measured as previously described. Each value represents the mean value of duplicate measurements (RLU x 1000).

| **Table 4B** | **1** | **2** | **3** | **4** | **5** |
|---|---|---|---|---|---|
| Serum A | 4,9 | 5,6 | 3,3 | 3,2 | 3,4 |
| Serum B | 63,3 | 76,1 | 60,3 | 25,4 | 63,2 |
| Mix A+B | 35,0 | 42,1 | 33,4 | 15,0 | 37,4 |

Table 4C: Stability experiments for the IGF1R autoantibodies assay. Serum with IGF1R autoantibodies was incubated for different time at 4°C and at room temperature (RT). Serum samples were analyzed in assay as described in example 5. Bound luciferase activity was measured as previously described. Each value represents the mean value of duplicate measurements (RLU x 1000).

| **Table 4C** | **0 days** | **5 days** | **10 days** |
|---|---|---|---|
| 4°C | 53,0 | 52,5 | 49,4 |
| RT | 53,0 | 52,2 | 53,8 |

### Example 6: Isolation of human IgG

1 ml of human serum was mixed with 1 ml PBS and 0.2 ml of protein G-sepharose and incubated overnight at 4°C by shaking. Protein G-sepharose was pelleted and washed ten times with PBS. Bound IgG were eluted with 25 mM citric acid, pH was adjusted to 7 using 1M Hepes-NaOH, pH 8. Eluted IgG were concentrated to 100 µl using Speedvac at room temperature. IgG was transferred into DMEM/F12 medium using speed filtration on sephadex G25 column.

### Example 7: Effect of autoantibodies on IGF1R autophosphorylation

HepG2 cells were seeded in 96 well plates (10 000 cells/well), incubated 24 h in complete DMEM/F12 medium followed by overnight incubation in serum free DMEM/F12 medium containing 0.1% BSA. After serum starvation cells were incubated with human IgG (10-20 mg/ml) in DMEM/F12 medium for 15 min up to 1h. After this in some cases IGF1 (1 ng/ml) was added and cells were incubated for the additional 15 min. Cells were washed with PBS containing phosphatase inhibitors and lysed in buffer 20 mM HEPES-NaOH pH 7.5, 50 mM NaCl, 2% triton X100, 10% glycerol, phosphatase inhibitors. Cell lysates were subjected to electrophoresis in 10% SDS PAGE and blotted to nitrocellulose membranes. Tyrosine phosphorylated IGF1R was detected on the blot using an anti-IGF1R (Tyr1165/Tyr1166) antibody. Bands were visualized using enhanced chemiluminescence western blotting detection kit (Amersham ECL Plus, GE Healthcare, General Electric Deutschland Holding GmbH, Frankfurt, Germany).

### Example 8: Effect of autoantibodies on cell growth

MCF7 cells were seeded at 2500 cells per well (96 well plates) and incubated overnight in complete DMEM/F12 medium. Cells were then starved in serum free DMEM/F12 medium containing 0.1% BSA for 5 h followed by addition of IgG (about 10 mg/ml) from five sera with low (1-5) and five sera with high (6-10) concentrations of autoantibodies against IGF1R (isolated from human patients), 1% FCS and 1 ng/ml IGF1. Cells were incubated for 5 days. The CellTiter-Glo luminescent cell viability kit was used to assess the number of viable cells in culture based on quantification of the ATP present, according to the manufacturer manual. Inhibiting effect of autoantibodies was expressed as Inhibition Index calculated as InI % = 100 x [(1 - (RLU test IgG) / (RLU negative IgG pool)]. Each value represents the mean value of duplicate measurements.

| **Table 5** | **Serum** | **Inhibition Index (%)** |
|---|---|---|
| | 1 | -7 |
| | 2 | 1 |
| | 3 | -3 |
| | 4 | -3 |
| | 5 | -2 |
| | 6 | 20 |
| | 7 | 18 |
| | 8 | 22 |
| | 9 | 16 |
| | 10 | 21 |

### Example 9: Bridge assays

### Example 9A: Bridge assay for the detection of autoantibodies interacting both with IGF1 receptor and insulin receptor.

Polystyrene tubes coated with PGA14 antibody were incubated overnight at 4°C with 200 µl of SF6 insect cell medium containing IGF1R-PGA14tag. After IGF1R-PGA14tag immobilization tubes were washed twice with 1ml of buffer 20 mM Tris-HCl pH 7.5, 50 mM NaCl, 10 % glycerol. Then each tube was incubated overnight at 4C° with a mixture of 100 µl of the same buffer containing 10 mg/ml BSA and 100 µl of a sample (serum probe). Tubes were washed twice with 1ml of the same buffer and incubated overnight at 4C° with 200 µl of IGF1R-Luc (or IR-Luc respectively) diluted in the same buffer with BSA (about 40x 10e6 RLU of luciferase activity). After incubation tubes were washed four times with 1ml of 20 mM Tris-HCl pH 7.5, 100 mM NaCl, 0.1 % triton X100. Finally, luciferase activity was measured in a Berthold luminometer (AutoLumat Plus LB 953) for 10 sec. Results were expressed as RLU (relative light units) bound. Each value represents the mean value of duplicate measurements.

| **Table 6** | **IGF1R-Luc** | **IGF1R-Luc** | **IR-Luc** | **IR-Luc** |
|---|---|---|---|---|
| **Bridge Assay** | **[RLU]** | **[% of max]** | **[RLU]** | **[% of max]** |
| without serum | 2135 | 5 | 947 | 5 |
| control serum | 5348 | 13 | 2616 | 15 |
| serum 406 | 42240 | 100 | 4814 | 27 |
| serum 522 | 39341 | 93 | 17961 | 100 |
| serum 531 | 36646 | 87 | 12839 | 71 |

### Example 9B: Detection limits of patient serum

An IGF1R-Ab positive serum was diluted with 20 mM Tris-HCl pH 7.5, 50 mM NaCl, 10% glycerol, 10 mg/ml BSA. The assay was performed as described in Example 9A. Negative serum is defined to give a signal like buffer control.

| **Table 7** | **Serum dilution (v/v)** | **IGF1R-Luc [RLU]** |
|---|---|---|
| | Undiluted | 64651 |
| | 1 / 4 | 38829 |
| | 1 / 16 | 20269 |
| | 1 / 64 | 8135 |
| | 1 / 256 | 5383 |
| | background (negative serum) | 5369 |

### Example 9C: Detection limits of commercially available anti-IGF1R antibodies

Monoclonal antibody to the extracellular domain of IGF1R (anti-IGF1R clone#24-75, Millipore, USA) was diluted with 20 mM Tris-HCl pH 7.5, 50 mM NaCl, 10% glycerol, 10 mg/ml BSA. The assay was performed as described in Example 9A.

| **Table 8** | **IGF1R-Luc** |
|---|---|
| **Antibody concentration (ng/ml)** | **[RLU]** |
| 50000 | 805261 |
| 12500 | 767157 |
| 3125 | 626879 |
| 781 | 475770 |
| 195 | 234773 |
| 49 | 88894 |
| 12 | 27013 |
| 3 | 11831 |
| 0.8 | 8856 |
| 0.2 | 5904 |
| 0.05 | 5369 |
| background | 5418 |

This result indicates that the detection limit of the assay according to the invention is about 0.2 ng/ml antibody concentration.

## Claims

1. A method for the identification of a modulator of the binding properties of analyte antibodies reactive with one or more antigenic molecules, said method comprising:
(a) providing one or more analyte antibodies reactive with one or more first and one or more second antigenic molecules; and
(b) providing one or more first antigenic molecules with which said analyte antibodies can interact and which first antigenic molecule is selected from the insulin receptor family (IRF); and
(c) providing one or more second antigenic molecules with which analyte antibodies can interact and which second antigenic molecule is selected from the IRF; and
(d) contacting said analyte antibodies as provided by step (a) and said first antigenic molecules as provided by step (b) and said second antigenic molecules as provided by step (c) simultaneously or successively with a sample to be investigated, whereby said modulator when present in said sample can interact with the said analyte antibodies and/or said antigenic molecules so as to interfere with the formation of complexes comprising [first antigenic molecule]-[analyte antibody]-[second antigenic molecule]; and
(e₁) prior to, or concurrent with, or subsequent to, step (d), providing immobilizing means whereby said first antigenic molecule as present in the said complexes formed in step (d), respectively, as capable to form complexes in step (d) can be immobilized to a solid support prior to, or concurrent with, or subsequent to, step (d); and/or
(e₂) prior to, or concurrent with, or subsequent to, step (d), providing second labeling means whereby said first antigenic molecule as present in the said complexes formed in step (d), respectively, as capable to form complexes in step (d) is labeled with said second labeling means prior to, or concurrent with, or subsequent to, step (d); and
(f) prior to, or concurrent with, or subsequent to, step (d), providing first labeling means whereby said second antigenic molecule as present in the said complexes formed in step (d), respectively, as capable to form complexes in step (d) is labeled with said first labeling means prior to, or concurrent with, or subsequent to, step (d); and
(g) prior to, or concurrent with, or subsequent to, step (d), providing one or more modulators capable to interact with the complexes formed in step (d) and/or capable to interfere with the complex formation according to step (d) and contacting said one or more modulators simultaneously or successively with the said sample, the said one or more first antigenic molecules, and/or the said one or more second antigenic molecules prior to, or concurrent with, or subsequent to step (d), or contacting said one or more modulators simultaneously or successively with the said complexes formed in or subsequent to step (d); and
(h) detecting the presence of complexes formed in or subsequent to step (d).

2. A method of detecting in a sample to be investigated the presence and/or the binding properties of analyte antibodies reactive with one or more antigenic molecules, said method comprising:
(a) providing one or more first antigenic molecules with which analyte antibodies when present in said sample can interact and which first antigenic molecule is selected from the insulin receptor family (IRF); and
(b) providing one or more second antigenic molecules with which analyte antibodies when present in said sample can interact and which second antigenic molecule is selected from the IRF; and
(c) contacting said first antigenic molecules as provided by step (a) and said second antigenic molecules as provided by step (b) simultaneously or successively with the sample to be investigated, whereby analyte antibodies when present in said sample can interact with said antigenic molecules so as to form complexes comprising [first antigenic molecule]-[analyte antibody]-[second antigenic molecule]; and
(d1) prior to, or concurrent with, or subsequent to, step (c), providing immobilizing means whereby said first antigenic molecule as present in the said complexes formed in step (c), respectively, as capable to form complexes in step (c) can be immobilized to a solid support prior to, or concurrent with, or subsequent to, step (c); and/or
(d2) prior to, or concurrent with, or subsequent to, step (c), providing second labeling means whereby said first antigenic molecule as present in the said complexes formed in step (c), respectively, as capable to form complexes in step (c) is labeled with said second labeling means prior to, or concurrent with, or subsequent to, step (c); and
(e) prior to, or concurrent with, or subsequent to, step (c), providing first labeling means whereby said second antigenic molecule as present in the said complexes formed in step (c), respectively, as capable to form complexes in step (c) is labeled with said first labeling means prior to, or concurrent with, or subsequent to, step (c); and
(g) detecting the presence of complexes formed in or subsequent to step (c) so as to provide indication of analyte antibodies present in said sample.

3. The method according to claim 2, further comprising:
(f) prior to, or concurrent with, or subsequent to, step (c), providing one or more modulators capable to interact with the complexes formed in step (c) and/or capable to interfere with the complex formation according to step (c) and contacting said one or more modulators simultaneously or successively with the said sample, the said one or more first antigenic molecules, and/or the said one or more second antigenic molecules prior to, or concurrent with, or subsequent to step (c), or contacting said one or more modulators simultaneously or successively with the said complexes formed in or subsequent to step (c).

4. The method according to one or more of claims 1 to 3, wherein said first antigenic molecules and said second antigenic molecules are identical.

5. The method according to one or more of claims 1 to 4, wherein said first antigenic molecules and/or said second antigenic molecules are embedded in a membrane environment.

6. The method according to one or more of claims 1 to 5, wherein said one or more first antigenic molecules and/or said one or more second antigenic molecules are lacking a functionally intact tyrosine kinase domain.

7. The method according to one or more of claims 1 to 6, wherein the said analyte antibody to be detected in said sample is an endogenous autoantibody or a monoclonal antibody.

8. The method according to one or more of claims 1 to 7, wherein one or more of the said means selected from the group consisting of said first labeling means, said second labeling means , said immobilization means, and said modulators are provided prior to the contacting said antigenic molecules and said analyte antibodies.

9. A kit useful for the performance of the method according to one ore more of claims 1 to 8 comprising
(a) one or more first antigenic molecules selected from the insulin receptor family as defined in one or more of claims 1 to 3 and 6;
(b) one or more second antigenic molecules selected from the insulin receptor family as defined in one or more of claims 1 to 3 and 6;
(c1) immobilization means as defined in claim 1 and/or
(c2) second labeling means as defined in claim 1; and
(d) first labeling means as defined in claim 1.

10. The kit according to claim 9 comprising
(a) the said first antigenic molecules are labeled with a second labeling means; and
(b) the said second antigenic molecules are labeled with a first labeling means.

11. The kit according to claim 9 comprising
(a) the said first antigenic molecules are immobilized to a solid support; and
(b) the said second antigenic molecules are labeled with a first labeling means.

12. The use of the method according to one ore more of claims 1 to 8 for the diagnosis of the presence or onset of a disease related to the insulin receptor family.

13. The use of the method according to one ore more of claims 1 to 8 for the identification of a pharmaceutically effective compound for the treatment and/or prophylaxis of a disease related to the insulin receptor family.

14. The use of a kit according to one or more of claims 9 to 11 for the diagnosis of the presence or onset of a disease related to the insulin receptor family.

15. The use of a kit according to one or more of claims 9 to 11 for the identification of a pharmaceutically effective compound for the treatment and/or prophylaxis of a disease related to the insulin receptor family.
